(19) **Europäisches Patentamt** / **European Patent Office** / **Office européen des brevets**

(11) **EP 2 830 488 B1**

(12) **EUROPEAN PATENT SPECIFICATION**

(45) Date of publication and mention
of the grant of the patent:
**15.08.2018 Bulletin 2018/33**

(21) Application number: **13872336.6**

(22) Date of filing: **13.03.2013**

(51) Int Cl.:
*A61M 5/158* (2006.01)       *A61B 5/00* (2006.01)
*A61M 5/168* (2006.01)

(86) International application number:
**PCT/US2013/031102**

(87) International publication number:
**WO 2014/116277 (31.07.2014 Gazette 2014/31)**

(54) **GEOMETRY OF A TRANSCUTANEOUS SENSOR**

GEOMETRIE EINES TRANSKUTANEN SENSORS

GÉOMÉTRIE D'UN CAPTEUR TRANSCUTANÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.01.2013 US 201361755273 P**

(43) Date of publication of application:
**04.02.2015 Bulletin 2015/06**

(73) Proprietor: **Ivwatch, LLC
Williamsburg, VA 23185 (US)**

(72) Inventors:
• **WARREN, Gary, P.
Williamsburg, VA 23185 (US)**
• **ALLEY, Matthew, S.
Sandston, VA 23150 (US)**
• **ANCHELL, Scott, J.
Fairfax Station, VA 22039 (US)**

• **NARAMORE, William, J.
Richmond, VA 23226 (US)**
• **BONNEMA, Garret, T.
Williamsburg, VA 23188 (US)**

(74) Representative: **Johnson, Richard Alan et al
Mewburn Ellis LLP
City Tower
40 Basinghall Street
London EC2V 5DE (GB)**

(56) References cited:
WO-A1-99/15074        WO-A2-96/41566
WO-A2-2006/116569     WO-A2-2010/053617
JP-A- H1 082 732      US-A- 4 877 034
US-A1- 2004 086 230   US-A1- 2005 014 997
US-A1- 2007 055 119   US-A1- 2009 163 836
US-B1- 7 826 890      US-B2- 7 546 776

## Description

TECHNICAL FIELD

The invention relates to a sensor to aid in diagnosing at least one of infiltration and extravasation in Animalia tissue.

BACKGROUND ART

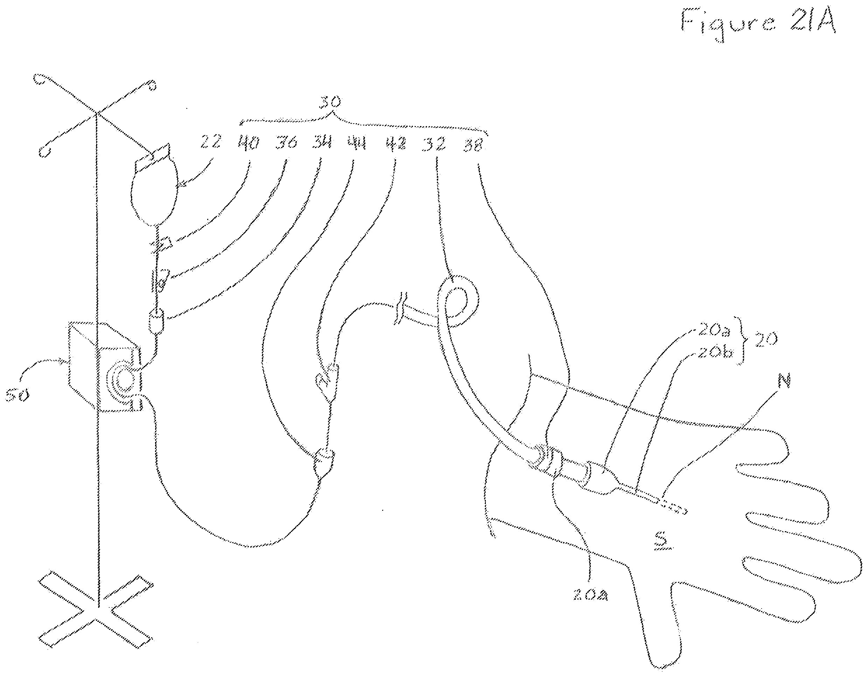

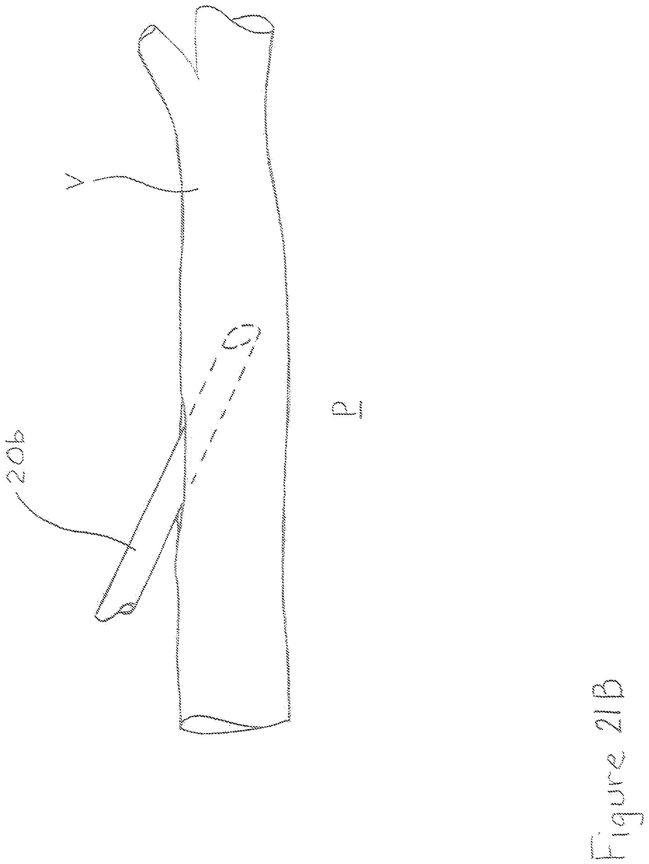

Figures 21A and 21B show a typical arrangement for intravascular infusion. As the terminology is used herein, "intravascular" preferably refers to being situated in, occurring in, or being administered by entry into a blood vessel, thus "intravascular infusion" preferably refers to introducing a fluid or infusate into a blood vessel. Intravascular infusion accordingly encompasses both intravenous infusion (administering a fluid into a vein) and intra-arterial infusion (administering a fluid into an artery).

A cannula 20 is typically used for administering fluid via a subcutaneous blood vessel V. Typically, cannula 20 is inserted through skin S at a cannulation or cannula insertion site N and punctures the blood vessel V, for example, the cephalic vein, basilica vein, median cubital vein, or any suitable vein for an intravenous infusion. Similarly, any suitable artery may be used for an intra-arterial infusion.

Cannula 20 typically is in fluid communication with a fluid source 22. Typically, cannula 20 includes an extracorporeal connector, e.g., a hub 20a, and a transcutaneous sleeve 20b. Fluid source 22 typically includes one or more sterile containers that hold the fluid(s) to be administered. Examples of typical sterile containers include plastic bags, glass bottles or plastic bottles.

An administration set 30 typically provides a sterile conduit for fluid to flow from fluid source 22 to cannula 20. Typically, administration set 30 includes tubing 32, a drip chamber 34, a flow control device 36, and a cannula connector 38. Tubing 32 is typically made of polypropylene, nylon, or another flexible, strong and inert material. Drip chamber 34 typically permits the fluid to flow one drop at a time for reducing air bubbles in the flow. Tubing 32 and drip chamber 34 are typically transparent or translucent to provide a visual indication of the flow. Typically, flow control device 36 is positioned upstream from drip chamber 34 for controlling fluid flow in tubing 32. Roller clamps and Dial-A-Flo®, manufactured by Hospira, Inc. (Lake Forest, Illinois, US), are examples of typical flow control devices. Typically, cannula connector 38 and hub 20a provide a leak-proof coupling through which the fluid may flow. Luer-Lok™, manufactured by Becton, Dickinson and Company (Franklin Lakes, New Jersey, US), is an example of a typical leak-proof coupling.

Administration set 30 may also include at least one of a clamp 40, an injection port 42, a filter 44, or other devices. Typically, clamp 40 pinches tubing 32 to cut-off fluid flow. Injection port 42 typically provides an access port for administering medicine or another fluid via cannula 20. Filter 44 typically purifies and/or treats the fluid flowing through administration set 30. For example, filter 44 may strain contaminants from the fluid.

An infusion pump 50 may be coupled with administration set 30 for controlling the quantity or the rate of fluid flow to cannula 20. The Alaris® System manufactured by CareFusion Corporation (San Diego, California, US), BodyGuard® Infusion Pumps manufactured by CMA America, L.L.C. (Golden, Colorado, US), and Flo-Gard® Volumetric Infusion Pumps manufactured by Baxter International Inc. (Deerfield, Illinois, US) are examples of typical infusion pumps.

Intravenous infusion or therapy typically uses a fluid (e.g., infusate, whole blood, or blood product) to correct an electrolyte imbalance, to deliver a medication, or to elevate a fluid level. Typical infusates predominately consist of sterile water with electrolytes (e.g., sodium, potassium, or chloride), calories (e.g., dextrose or total parenteral nutrition), or medications (e.g., anti-infectives, anticonvulsants, antihyperuricemic agents, cardiovascular agents, central nervous system agents, chemotherapy drugs, coagulation modifiers, gastrointestinal agents, or respiratory agents). Examples of medications that are typically administered during intravenous therapy include acyclovir, allopurinol, amikacin, aminophylline, amiodarone, amphotericin B, ampicillin, carboplatin, cefazolin, cefotaxime, cefuroxime, ciprofloxacin, cisplatin, clindamycin, cyclophosphamide, diazepam, docetaxel, dopamine, doxorubicin, doxycycline, erythromycin, etoposide, fentanyl, fluorouracil, furosemide, ganciclovir, gemcitabine, gentamicin, heparin, imipenem, irinotecan, lorazepam, magnesium sulfate, meropenem, methotrexate, methylprednisolone, midazolam, morphine, nafcillin, ondansetron, paclitaxel, pentamidine, phenobarbital, phenytoin, piperacillin, promethazine, sodium bicarbonate, ticarcillin, tobramycin, topotecan, vancomycin, vinblastine and vincristine. Transfusions and other processes for donating and receiving whole blood or blood products (e.g., albumin and immunoglobulin) also typically use intravenous infusion.

Unintended infusing typically occurs when fluid from cannula 20 escapes from its intended vein/artery. Typically, unintended infusing causes an abnormal amount of the fluid to diffuse or accumulate in perivascular tissue P and may occur, for example, when (i) cannula 20 causes a vein/artery to rupture; (ii) cannula 20 improperly punctures the vein/artery; (iii) cannula 20 backs out of the vein/artery; (iv) cannula 20 is improperly sized; (v) infusion pump 50 administers fluid at an excessive flow rate; or (vi) the infusate increases permeability of the vein/artery. As the terminology is used herein, "tissue" preferably refers to an association of cells, intercellular material and/or interstitial compartments, and "perivascular tissue" preferably refers to cells, intercellular material and/or interstitial compartments that are in the general vicinity of a blood vessel and may become unintentionally infused with fluid from cannula 20. Unintended infusing of a non-vesicant fluid is typically referred to as "infiltration," whereas unintended infusing of a vesicant fluid is typically referred to as "extravasation."

The symptoms of infiltration or extravasation typically include blanching or discoloration of the skin S, edema, pain, or numbness. The consequences of infiltration or extravasation typically include skin reactions (e.g., blisters), nerve compression, compartment syndrome, or necrosis. Typical treatment for infiltration or extravasation includes applying warm or cold compresses, elevating an affected limb, administering hyaluronidase, phentolamine, sodium thiosulfate or dexrazoxane, fasciotomy, or amputation.

US 2004/0086230 A1 discloses a sensor with the features of the preamble of claim 1 and a method of manufacturing such a sensor with the features of the preamble of claim 4. It discloses a light mixer for use in connection with an optical probe of a spectrophotometric-type instrument. The mixer combines different wavelength light beams from a plurality of discrete optical fibers in a homogeneous beam before transmission into biological tissue.

DISCLOSURE OF INVENTION

According to one aspect of the present invention, there is provided a sensor to aid in diagnosing at least one of infiltration and extravasation in Animalia tissue as set out in claim 1.

According to another aspect of the present invention, there is provided a method of manufacturing a sensor to aid in diagnosing at least one of infiltration and extravasation in Animalia tissue as set out in claim 4.

BRIEF DESCRIPTION OF DRAWINGS

The accompanying drawings, together with the general description given above and the detailed description given below, serve to explain the features, principles, and methods of the invention.

Figure 1 is a schematic view illustrating an electromagnetic radiation sensor according to the present disclosure. The electromagnetic radiation sensor is shown contiguously engaging Animalia skin.

Figures 2A-2C are schematic cross-section views demonstrating how an anatomical change over time in perivascular tissue impacts the electromagnetic radiation sensor shown in Figure 1.

Figure 3 is a schematic exploded cross-section view of the electromagnetic radiation sensor shown in Figure 1.

Figure 4 is a schematic plan view illustrating a superficies geometry of the electromagnetic radiation sensor shown in Figure 1.

Figures 5A-5C are schematic cross-section views demonstrating the impact of different nominal spacing distances between emission and detection waveguides of the electromagnetic radiation sensor shown in Figure 1.

Figure 6 is a graph illustrating a relationship between spacing, depth and wavelength for the electromagnetic radiation sensor shown in Figure 1.

Figure 7 illustrates a technique for developing the superficies shown in Figure 4.

Figure 8 is a schematic plan view illustrating another superficies geometry according to the present disclosure.

Figure 9 is a schematic plan view illustrating several variations of another superficies geometry according to the present disclosure.

Figure 10 is a schematic plan view illustrating another superficies geometry according to the present disclosure.

Figure 11 is a schematic plan view illustrating another superficies geometry according to the present disclosure.

Figure 12 is a schematic plan view illustrating another superficies geometry according to the present disclosure.

Figure 13 is a schematic plan view illustrating several variations of another superficies geometry according to the present disclosure.

Figures 14A-14D illustrate distributions of spacing distances for examples of superficies geometries according to the present disclosure.

Figures 15-18 are schematic cross-section views illustrating topographies of superficies geometries according to the present disclosure.

Figure 19 is a schematic cross-section view illustrating an angular relationship between waveguides of the electromagnetic radiation sensor shown in Figure 1.

Figure 20A is a schematic cross-section view illustrating another angular relationship between waveguides of an electromagnetic radiation sensor according to the present disclosure.

Figure 20B illustrates a technique for representing the interplay between emitted and collected radiation of the waveguides shown in Figure 20A.

Figure 21A is a schematic view illustrating a typical set-up for infusion administration.

Figure 21B is a schematic view illustrating a subcutaneous detail of the set-up shown in Figure 21A.

In the figures, the thickness and configuration of components may be exaggerated for clarity. The same reference numerals in different figures represent the same component.

BEST MODE(S) FOR CARRYING OUT THE INVENTION

The following description and drawings are illustrative and are not to be construed as limiting. Numerous specific details are described to provide a thorough understanding of the disclosure. However, in certain instances, well-known or conventional details are not described in order to avoid obscuring the description.

Reference in this specification to "one embodiment" or "an embodiment" means that a particular feature, structure, or characteristic described in connection with the embodiment is included in at least one embodiment according to the disclosure. The appearances of the phrases "one embodiment" or "other embodiments" in various places in the specification are not necessarily all referring to the same embodiment, nor are separate or alternative embodiments mutually exclusive of other embodiments. Moreover, various features are described that may be exhibited by some embodiments and not by others. Similarly, various features are described that may be included in some embodiments but not other embodiments.

The terms used in this specification generally have their ordinary meanings in the art, within the context of the disclosure, and in the specific context where each term is used. Certain terms in this specification may be used to provide additional guidance regarding the description of the disclosure. It will be appreciated that a feature may be described more than one-way.

Alternative language and synonyms may be used for any one or more of the terms discussed herein. No special significance is to be placed upon whether or not a term is elaborated or discussed herein. Synonyms for certain terms are provided. A recital of one or more synonyms does not exclude the use of other synonyms. The use of examples anywhere in this specification including examples of any terms discussed herein is illustrative only, and is not intended to further limit the scope and meaning of the disclosure or of any exemplified term.

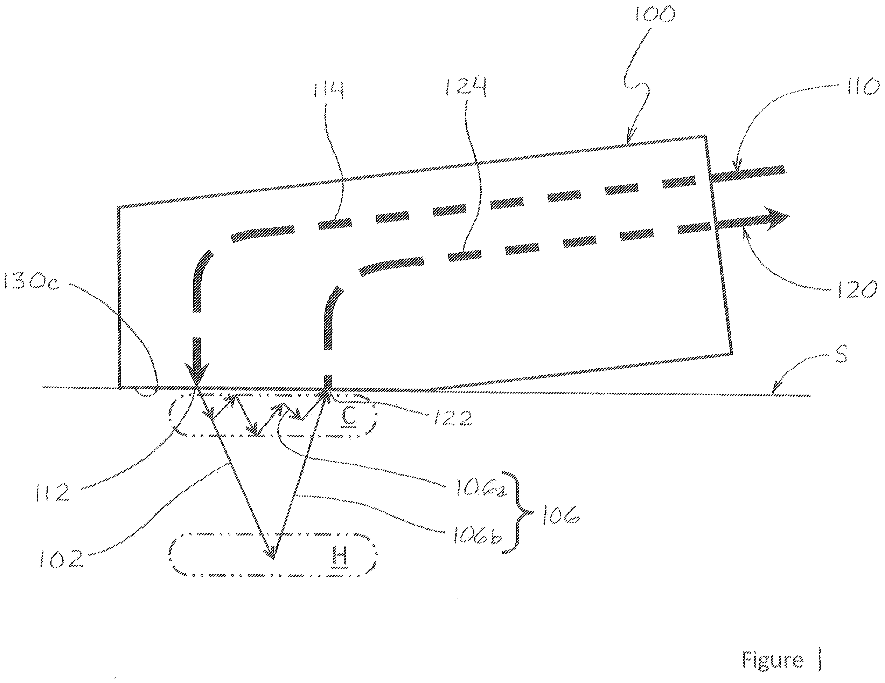

Figure 1 shows an electromagnetic radiation sensor 100 that preferably includes an anatomic sensor. As the terminology is used herein, "anatomic" preferably refers to the structure of an Animalia body and an "anatomic sensor" preferably is concerned with sensing a change over time of the structure of the Animalia body. By comparison, a physiological sensor is concerned with sensing the functions or activities of an Animalia body, e.g., pulse or blood chemistry, at a point in time.

Electromagnetic radiation sensor 100 preferably is coupled with the skin S. Preferably, electromagnetic radiation sensor 100 is arranged to overlie a target area of the skin S. As the terminology is used herein, "target area" preferably refers to a portion of a patient's skin that is generally proximal to where an infusate is being administered and frequently proximal to the cannulation site N. Preferably, the target area overlies the perivascular tissue P. According to one embodiment, adhesion preferably is used to couple electromagnetic radiation sensor 100 to the skin S. According to other embodiments, any suitable coupling may be used that preferably minimizes relative movement between electromagnetic radiation sensor 100 and the skin S.

Electromagnetic radiation sensor 100 preferably emits and collects transcutaneous electromagnetic radiation signals, e.g., light signals. Preferably, electromagnetic radiation sensor 100 emits electromagnetic radiation 102 and collects electromagnetic radiation 106. Emitted electromagnetic radiation 102 preferably passes through the target area of the skin S toward the perivascular tissue P. Collected electromagnetic radiation 106 preferably includes a portion of emitted electromagnetic radiation 102 that is at least one of specularly reflected, diffusely reflected (e.g., due to elastic or inelastic scattering), fluoresced (e.g., due to endogenous or exogenous factors), or otherwise redirected from the perivascular tissue P before passing through the target area of the skin S.

Electromagnetic radiation sensor 100 preferably includes waveguides to transmit emitted and collected electromagnetic radiation 102 and 106. As the terminology is used herein, "waveguide" preferably refers to a duct, pipe, fiber, or other device that generally confines and directs the propagation of electromagnetic radiation along a path. Preferably, an emission waveguide 110 includes an emitter face 112 for emitting electromagnetic radiation 102 and a detection waveguide 120 includes a detector face 122 for collecting electromagnetic radiation 106. According to one embodiment, emission waveguide 110 preferably includes a set of emission optical fibers 114 and detection waveguide 120 preferably includes a set of detection optical fibers 124. Individual emission and detection optical fibers 114 and 124 preferably each have an end face. Preferably, an aggregation of end faces of emission optical fibers 114 forms emitter face 112 and an aggregation of end faces of detection optical fibers 124 forms detector face 122.

The transcutaneous electromagnetic radiation signals emitted by electromagnetic radiation sensor 100 preferably are not harmful to an Animalia body. Preferably, the wavelength of emitted electromagnetic radiation 102 is longer than at least approximately 400 nanometers. The frequency of emitted electromagnetic radiation 102 therefore is no more than approximately 750 terahertz. According to one embodiment, emitted electromagnetic radiation 102 is in the visible radiation (light) or infrared radiation portions of the electromagnetic spectrum. Preferably, emitted electromagnetic radiation 102 is in the near infrared portion of the electromagnetic spectrum. As the terminology is used herein, "near infrared" preferably refers to electromagnetic radiation having wavelengths between approximately 600 nanometers and approximately 2,100 nanometers. These wavelengths correspond to a frequency range of approximately 500 terahertz to approximately 145 terahertz. A desirable range in the near infrared portion of the electromagnetic spectrum preferably includes wavelengths between approximately 800 nanometers and approximately 1,050 nanometers. These wavelengths correspond to a frequency range of approximately 375 terahertz to approximately 285 terahertz. According to other embodiments, electromagnetic radiation sensor 100 may emit electromagnetic radiation signals in shorter wavelength portions of the electromagnetic spectrum, e.g., ultraviolet light, X-rays or gamma rays, preferably when radiation intensity and/or signal duration are such that tissue harm is minimized.

Emitted and collected electromagnetic radiation 102 and 106 preferably share one or more wavelengths. According to one embodiment, emitted and collected electromagnetic radiation 102 and 106 preferably share a single peak wavelength, e.g., approximately 940 nanometers (approximately 320 terahertz). As the terminology is used herein, "peak wavelength" preferably refers to an interval of wavelengths including a spectral line of peak power. The interval preferably includes wavelengths having at least half of the peak power. Preferably, the wavelength interval is +/- approximately 20 nanometers with respect to the spectral line. According to other embodiments, emitted and collected electromagnetic radiation 102 and 106 preferably share a plurality of peak wavelengths, e.g., approximately 940 nanometers and approximately 650 nanometers (approximately 460 terahertz). According to other embodiments, a first one of emitted and collected electromagnetic radiation 102 and 106 preferably spans a first range of wavelengths, e.g., from approximately 600 nanometers to approximately 1000 nanometers. This wavelength range corresponds to a frequency range from approximately 500 terahertz to approximately 300 terahertz. A second one of emitted and collected electromagnetic radiation 102 and 106 preferably shares with the first range a single peak wavelength, a plurality of peak wavelengths, or a second range of wavelengths. Preferably, an optical power analysis at the wavelength(s) shared by emitted and collected electromagnetic radiation 102 and 106 provides an indication of anatomical change over time in the perivascular tissue P.

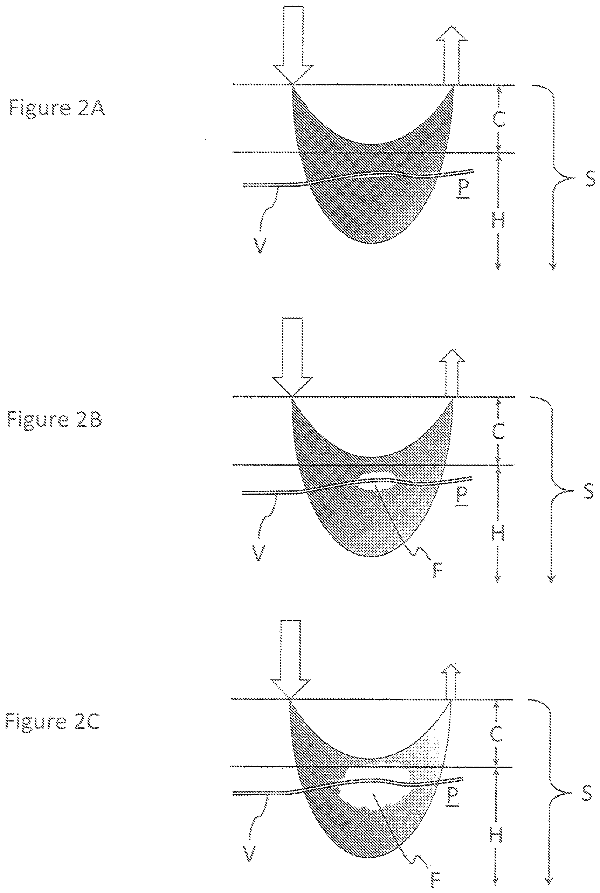

Figures 2A-2C schematically illustrate how an infiltration/extravasation event preferably evolves. Figure 2A shows the skin S prior to an infiltration/extravasation event. Preferably, the skin S includes cutaneous tissue C, e.g., stratum corneum, epidermis and/or dermis, overlying subcutaneous tissue, e.g., hypodermis H. Blood vessels V suitable for intravenous therapy typically are disposed in the hypodermis H. Figure 2B shows an infusate F beginning to accumulate in the perivascular tissue P. Accumulation of the infusate F typically begins in the hypodermis H, but may also begin in the cutaneous tissue C or at an interface of the hypodermis H with the cutaneous tissue C. Figure 2C shows additional accumulation of the infusate F in the perivascular tissue P. Typically, the additional accumulation extends further in the hypodermis H but may also extend into the cutaneous tissue C. According to one embodiment, an infiltration/extravasation event generally originates and/or occurs in proximity to the blood vessel V, e.g., as illustrated in Figures 2A-2C. According to other embodiments, an infiltration/extravasation event may originate and/or occur some distance from the blood vessel V, e.g., if pulling on the cannula C or administration set 30 causes the cannula outlet to become displaced from the blood vessel V.

Figures 2A-2C also schematically illustrate the relative power of emitted and collected electromagnetic radiation 102 and 106. Preferably, emitted electromagnetic radiation 102 enters the skin S, electromagnetic radiation propagates through the skin S, and collected electromagnetic radiation 106 exits the skin S. Emitted electromagnetic radiation 102 is schematically illustrated with an arrow directed toward the skin S and collected electromagnetic radiation 106 is schematically illustrated with an arrow directed away from the skin S. Preferably, the relative sizes of the arrows correspond to the relative powers of emitted and collected electromagnetic radiation 102 and 106. The propagation is schematically illustrated with crescent shapes that preferably include the predominant electromagnetic radiation paths through the skin S from emitted electromagnetic radiation 102 to collected electromagnetic radiation 106. Stippling in the crescent shapes schematically illustrates a distribution of electromagnetic radiation power in the skin S with relatively lower power generally indicated with less dense stippling and relatively higher power generally indicated with denser stippling.

The power of collected electromagnetic radiation 106 preferably is impacted by the infusate F accumulating in the perivascular tissue P. Prior to the infiltration/extravasation event (Figure 2A), the power of collected electromagnetic radiation 106 preferably is a fraction of the power of emitted electromagnetic radiation 102 due to electromagnetic radiation scattering and absorption by the skin S. Preferably, the power of collected electromagnetic radiation 106 changes with respect to emitted electromagnetic radiation 102 in response to the infusate F accumulating in the perivascular tissue P (Figures 2B and 2C). According to one embodiment, emitted and collected electromagnetic radiation 102 and 106 include near infrared electromagnetic radiation. The power of collected electromagnetic radiation 106 preferably decreases due to scattering and/or absorption of near infrared electromagnetic radiation by the infusate F. The compositions of most infusates typically are dominated by water. Typically, water has different absorption and scattering coefficients as compared to the perivascular tissue P, which contains relatively strong near infrared energy absorbers, e.g., blood. At wavelengths shorter than approximately 700 nanometers (approximately 430 terahertz), absorption coefficient changes preferably dominate due to absorption peaks of blood. Preferably, scattering coefficient changes have a stronger influence than absorption coefficient changes for wavelengths between approximately 800 nanometers (approximately 375 terahertz) and approximately 1,300 nanometers (approximately 230 terahertz). In particular, propagation of near infrared electromagnetic radiation in this range preferably is dominated by scattering rather than absorption because scattering coefficients have a larger magnitude than absorption coefficients. Absorption coefficient changes preferably dominate between approximately 1,300 nanometers and approximately 1,500 nanometers (approximately 200 terahertz) due to absorption peaks of water. Therefore, the scattering and/or absorption impact of the infusate F accumulating in the perivascular tissue P preferably is a drop in the power signal of collected electromagnetic radiation 106 relative to emitted electromagnetic radiation 102. According to other embodiments, a rise in the power signal of collected electromagnetic radiation 106 relative to emitted electromagnetic radiation 102 preferably is related to infusates with different scattering and absorption coefficients accumulating in the perivascular tissue P. Thus, the inventors discovered, *inter alia,* that fluid changes in perivascular tissue P over time, e.g., due to an infiltration/extravasation event, preferably are indicated by a change in the power signal of collected electromagnetic radiation 106 with respect to emitted electromagnetic radiation 102.

Electromagnetic radiation sensor 100 preferably aids healthcare givers in identifying infiltration/extravasation events. Preferably, changes in the power signal of collected electromagnetic radiation 106 with respect to emitted electromagnetic radiation 102 alert a healthcare giver to perform an infiltration/extravasation evaluation. The evaluation that healthcare givers perform to identify infiltration/extravasation events typically includes palpitating the skin S in the vicinity of the target area, observing the skin S in the vicinity of the target area, and/or comparing limbs that include and do not include the target area of the skin S.

The inventors discovered a problem regarding accurately alerting healthcare givers to perform an infiltration/extravasation evaluation. In particular, healthcare givers may not be accurately alerted because of a relatively low signal-to-noise ratio of collected electromagnetic radiation 106. Thus, the inventors discovered, *inter alia,* that noise in collected electromagnetic radiation 106 frequently obscures signals that alert healthcare givers to perform an infiltration/extravasation evaluation.

The inventors also discovered a source of the problem is emitted electromagnetic radiation 102 being reflected, scattered, or otherwise redirected from various tissues/depths below the stratum corneum of the skin S. Referring again to Figure 1, the inventors discovered that a first portion 106a of collected electromagnetic radiation 106 includes emitted electromagnetic radiation 102 that is reflected, scattered, or otherwise redirected from relatively shallow tissue, e.g., the cutaneous tissue C, and that a second portion 106b of collected electromagnetic radiation 106 includes emitted electromagnetic radiation 102 that is reflected, scattered, or otherwise redirected from the relatively deep tissue, e.g., the hypodermis H. The inventors further discovered, *inter alia,* that second portion 106b from relatively deep tissue includes a signal that more accurately alerts healthcare givers to perform an infiltration/extravasation evaluation and that first portion 106a from relatively shallow tissue includes noise that frequently obscures the signal in second portion 106b.

The inventors further discovered that sensor configuration preferably is related to the signal-to-noise ratio of a skin-coupled sensor. In particular, the inventors discovered that the relative configuration of emission and detection waveguides 110 and 120 preferably impact the signal-to-noise ratio of electromagnetic radiation sensor 100. Thus, the inventors discovered, *inter alia,* that the geometry, topography and/or angles of emission and detection waveguides 110 and 120 preferably impact the sensitivity of electromagnetic radiation sensor 100 to the signal in second portion 106b relative to the noise in first portion 106a.

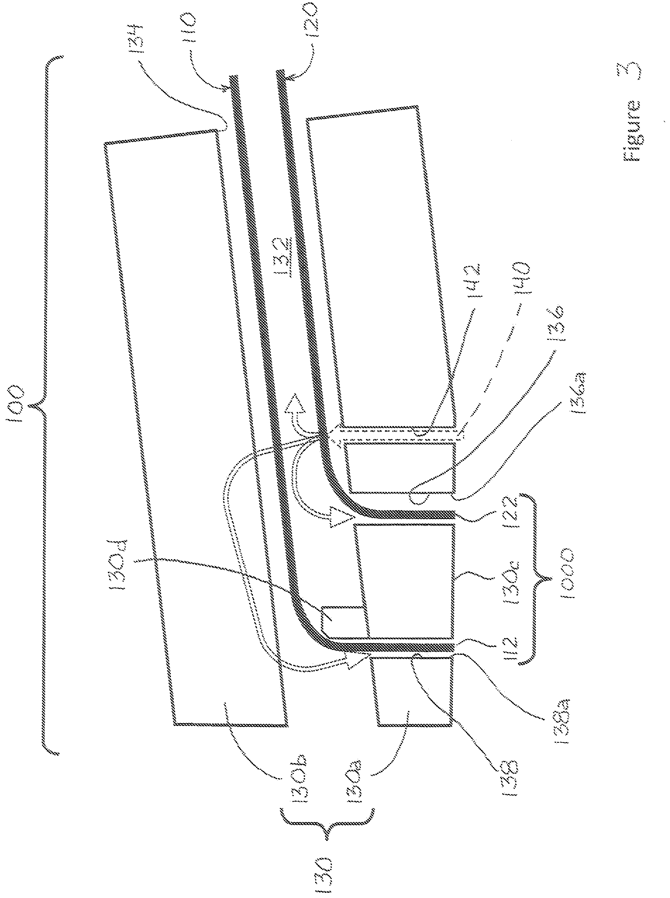

Figure 3 is an exploded schematic cross-section view illustrating the relative configuration between emission and detection waveguides 110 and 120 with respect to a housing 130 of electromagnetic radiation sensor 100. Preferably, the housing 130 includes a first housing portion 130a and a second housing portion 130b. The first and second housing portions 130a and 130b preferably are at least one of adhered, welded, interference fitted or otherwise coupled so as to define an internal volume 132. Internal volume 132 preferably extends between first and second ends. Preferably, an entrance 134 is disposed at the first end of internal volume 132 and sets of passages through first housing portion 130a are disposed at the second end of internal volume 132. Entrance 134 preferably provides emission and detection waveguides 110 and 120 with mutual access to internal volume 132. Preferably, a set of emission passages 136 provides emission waveguide 110 with individual egress from internal volume 132, and a set of detection passages 138 provides detection waveguide 120 with individual egress from internal volume 132. Accordingly, sets of emission and detection passages 136 and 138 preferably separate emission waveguide 110 with respect to detection waveguide 120. Preferably, emission passages 136 include emission apertures 136a that penetrate surface 130c, and detection passages 138 include detection apertures 138a that penetrate surface 130c. According to one embodiment, at least one of first and second housing portions 130a and 130b preferably includes an internal wall 130d for supporting, positioning and/or orienting at least one of emission and detection waveguides 110 and 120 in internal volume 132. According to other embodiments, at least first housing portion 130a preferably includes a substantially biocompatible material, e.g., polycarbonate.

Electromagnetic radiation sensor 100 preferably is positioned in close proximity to the skin S. As the terminology is used herein, "close proximity" of electromagnetic radiation sensor 100 with respect to the skin S preferably refers to a relative arrangement that minimizes gaps between a surface 130c of first housing portion 130a and the stratum corneum of the skin S. Preferably, surface 130c confronts the stratum corneum of the skin S. According to one embodiment, surface 130c preferably contiguously engages the skin S. (See, for example, Figure 1.) According to other embodiments, a film (not shown) that is suitably transparent to electromagnetic radiation preferably is interposed between surface 130c and the skin S.

A filler 140 preferably fixes the relative configuration of emission and detection waveguides 110 and 120 in housing 130. Preferably, filler 140 is injected under pressure via a fill hole 142 so as to occupy voids in internal volume 132 and to substantially cincture emission and detection waveguides 110 and 120. For example, filler 140 preferably occupies voids between (i) emission waveguide 110 and first housing portion 130a, including emission passages 136; (ii) emission waveguide 110 and second housing portion 130b; (iii) detection waveguide 120 and first housing portion 130a, including detection passages 138; (iv) detection waveguide 120 and second housing portion 130b; and (v) emission waveguides 110 and 120. Preferably, filler 140 extends at least as far as entrance 134, emission apertures 136a, and detection apertures 138a. Filler 140 preferably includes epoxy or another adhesive that is injected as an uncured liquid and subsequently cures as a solid. Thus, filler 140 preferably substantially fixes the relative positions/orientations of housing 130, emission waveguide 110, and detection waveguide 120. According to one embodiment, filler 140 preferably couples first and second housing portions 130a and 130b. According to other embodiments, filler 140 preferably includes first and second components. Preferably, the first component of filler 140 fastens at least one of emission and detection waveguides 110 and 120 with respect to first housing portion 130a and the second component of filler 140 packs internal volume 132. The first and second components of filler 140 preferably are sequentially introduced to internal volume 132. According to other embodiments, filler 140 preferably includes an electromagnetic radiation absorbing material.

Electromagnetic radiation sensor 100 preferably includes a superficies 1000 that overlies the skin S. Preferably, superficies 1000 includes surface 130c, emitter face 112, and detector face 122. Superficies 1000 preferably may also include façades of filler 140 that occlude emission and detection apertures 136a and 138a around emitter and detector end faces 112 and 122. Preferably, superficies 1000 is a three-dimensional surface contour that is generally smooth. As the terminology is used herein, "smooth" preferably refers to being substantially continuous and free of abrupt changes.

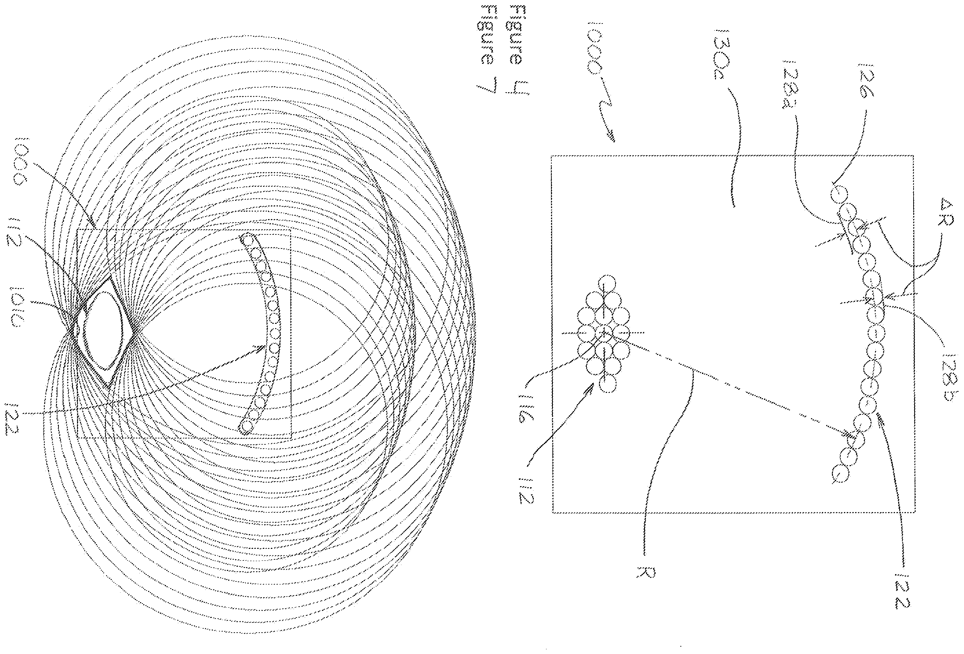

Figure 4 shows an example of superficies 1000 having a suitable geometry for observing anatomical changes over time in the perivascular tissue P. In particular, the geometry of superficies 1000 preferably includes the relative spacing and shapes of emitter and detector faces 112 and 122. According to one embodiment, a cluster of emission optical fiber end faces preferably has a geometric centroid 116 and an arcuate arrangement of detection optical fiber end faces preferably extends along a curve 126. As the terminology is used herein, "cluster" preferably refers to a plurality of generally circular optical fiber end faces that are arranged such that at least one end face is approximately tangent with respect to at least three other end faces. Preferably, curve 126 has a radius of curvature R that extends from an origin substantially coincident with geometric centroid 116. Curve 126 may be approximated by a series of line segments that correspond to individual chords of generally circular detection optical fiber end faces. Accordingly, each detection optical fiber end face preferably is tangent to at most two other end faces. The arcuate arrangement of detection optical fiber end faces preferably includes borders with radii of curvature that originate at geometric centroid 116, e.g., similar to curve 126. Preferably, a concave border 128a has a radius of curvature that is less than the radius of curvature R by an increment ΔR, and a convex border 128b has a radius of curvature that is greater than the radius of curvature R by an increment ΔR. According to one embodiment, increment ΔR is approximately equal to the radius of individual detection optical fiber end faces. According to other embodiments, detector face 122 preferably includes individual sets of detection optical fiber end faces arranged in generally concentric curves disposed in a band between concave and convex borders 128a and 128b. As the terminology is used herein, "band" preferably refers to a strip or stripe that is differentiable from an adjacent area or material.

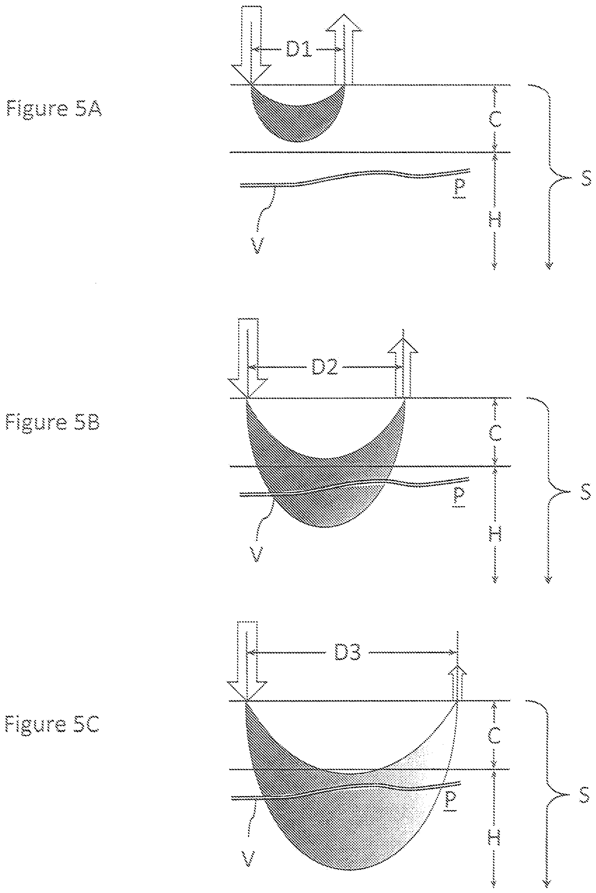

Figures 5A-5C illustrate how different nominal spacing distances between emission and detection waveguides 110 and 120 preferably impact collected electromagnetic radiation 106. Preferably, emitted electromagnetic radiation 102 enters the skin S from emission waveguide 110, electromagnetic radiation propagates through the skin S, and collected electromagnetic radiation 106 exits the skin S to detection waveguide 120. Emitted electromagnetic radiation 102 is schematically illustrated with an arrow directed toward the skin S and collected electromagnetic radiation 106 is schematically illustrated with an arrow directed away from the skin S. Preferably, the relative sizes of the arrows correspond to the relative powers of emitted and collected electromagnetic radiation 102 and 106. Electromagnetic radiation in the near infrared portion of the electromagnetic spectrum preferably is measured in milliwatts, decibel milliwatts or another unit suitable for indicating optical power. The propagation is schematically illustrated with crescent shapes that preferably include the predominant electromagnetic radiation paths through the skin S from emitted electromagnetic radiation 102 to collected electromagnetic radiation 106. Stippling in the crescent shapes schematically illustrates a distribution of electromagnetic radiation power in the skin S with relatively lower power generally indicated with less dense stippling and relatively higher power generally indicated with denser stippling. Referring to Figure 5A, a first nominal spacing distance D1 preferably separates emitted electromagnetic radiation 102 and collected electromagnetic radiation 106. At the first nominal spacing distance D1, the paths of electromagnetic radiation through the skin S generally are relatively short and predominantly extend through the cutaneous tissue C. Referring to Figure 5B, a second nominal spacing distance D2 preferably separates emitted electromagnetic radiation 102 and collected electromagnetic radiation 106. At the second nominal spacing distance D2, the paths of electromagnetic radiation preferably penetrate deeper into the skin S and extend in both the cutaneous tissue C and the hypodermis H. Referring to Figure 5C, a third nominal spacing distance D3 preferably separates emitted electromagnetic radiation 102 and collected electromagnetic radiation 106. At the third nominal spacing distance D3, the paths of electromagnetic radiation through the skin S generally are relatively long and predominantly extend through the hypodermis H.

The inventors discovered, *inter alia,* that varying the spacing distance between emission and detection waveguides 110 and 120 preferably changes a balance between the power and the signal-to-noise ratio of collected electromagnetic radiation 106. The relative power of collected electromagnetic radiation 106 with respect to emitted electromagnetic radiation 102 preferably is greater for narrower nominal spacing distance D1 as compared to broader nominal spacing distance D3. On the other hand, the signal-to-noise ratio of collected electromagnetic radiation 106 preferably is higher for broader nominal spacing distance D3 as compared to narrower nominal spacing distance D1. Preferably, there is an intermediate nominal spacing distance D2 that improves the signal-to-noise ratio as compared to narrower nominal spacing distance D1 and, as compared to broader nominal spacing distance D3, improves the relative power of collected electromagnetic radiation 106 with respect to emitted electromagnetic radiation 102.

The inventors designed and analyzed a skin phantom preferably to identify an optimum range for the intermediate nominal spacing distance D2. Preferably, the skin phantom characterizes several layers of Animalia skin including at least the epidermis (including the stratum corneum), dermis, and hypodermis. Table A shows the thicknesses, refractive indices, scattering coefficients, and absorption coefficients for each layer according to one embodiment of the skin phantom. Analyzing the skin phantom preferably includes tracing the propagation of up to 200,000,000 or more rays through the skin phantom to predict changes in the power of collected electromagnetic radiation 106. Examples of suitable ray-tracing computer software include ASAP® from Breault Research Organization, Inc. (Tucson, Arizona, US) and an open source implementation of a Monte Carlo Multi-Layer (MCML) simulator from the Biophotonics Group at the Division of Atomic Physics (Lund University, Lund, SE). The MCML simulator preferably uses CUDA™ from NVIDIA Corporation (Santa Clara, California, US) or another parallel computing platform and programming model. Preferably, a series of 1-millimeter thick sections simulate infiltrated perivascular tissue at depths up to 10 millimeters below the stratum corneum. The infiltrated perivascular tissue sections preferably are simulated with an infusate that approximates water, e.g., having a refractive index of approximately 1.33. Based on computer analysis of the skin phantom, the inventors discovered, *inter alia,* a relationship exists between (1) the spacing distance between emission and detection waveguides 110 and 120; (2) an expected depth below the stratum corneum for the perivascular tissue P at which anatomical changes over time preferably are readily observed; and (3) the wavelength of the electromagnetic radiation.

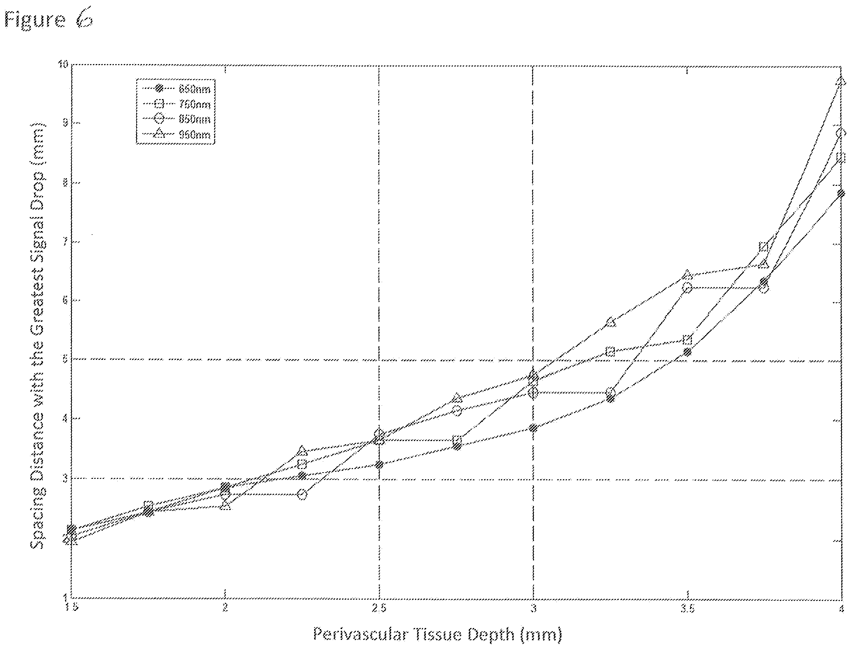

Figure 6 shows a graphical representation of the spacing/depth/wavelength relationship based on a computer analysis of the skin phantom. In particular, Figure 6 shows a plot of spacing distances with the greatest signal drop at various perivascular tissue depths for certain wavelengths of electromagnetic radiation. The terminology "spacing distance with the greatest signal drop" preferably refers to the spacing distance between emission and detection waveguides 110 and 120 that experiences the greatest drop in the power signal of collected electromagnetic radiation 106. The terminology "perivascular tissue depth" preferably refers to the depth below the stratum corneum of the perivascular tissue P at which anatomical changes over time are readily observed. According to the invention illustrated in Figure 6, emission and detection waveguides 110 and 120 that preferably are separated between approximately 3 millimeters and approximately 5 millimeters are expected to readily observe anatomical changes at depths between approximately 2.5 millimeters and approximately 3 millimeters below the stratum corneum for wavelengths between approximately 650 nanometers and approximately 950 nanometers (between approximately 460 terahertz and approximately 315 terahertz). Preferably, the spacing distance range between emission and detection waveguides 110 and 120 is between approximately 3.7 millimeters and approximately 4.4 millimeters to observe an anatomical change over time in the perivascular tissue P at an expected depth of approximately 2.75 millimeters when the electromagnetic radiation wavelength is between approximately 650 nanometers and approximately 950 nanometers. The spacing distance between emission and detection waveguides 110 and 120 preferably is approximately 4.5 millimeters to observe an anatomical change over time in the perivascular tissue P at an expected depth of approximately 2.8 millimeters when the electromagnetic radiation wavelength is approximately 950 nanometers. Preferably, the spacing distance between emission and detection waveguides 110 and 120 is approximately 4 millimeters to observe an anatomical change over time in the perivascular tissue P at an expected depth of approximately 2.6 millimeters when the electromagnetic radiation wavelength is between approximately 850 nanometers (approximately 350 terahertz) and approximately 950 nanometers.

Electromagnetic radiation sensor 100 preferably aids in observing anatomical changes that also occur at unexpected depths below the stratum corneum of the skin S. Preferably, the expected depth at which an anatomical change is expected to occur is related to, for example, the thickness of the cutaneous tissue C and the location of blood vessels V in the hypodermis H. Relatively thicker cutaneous tissue C and/or a blood vessel V located relatively deeper in the hypodermis H preferably increase the expected perivascular tissue depth for readily observing an anatomical change. Conversely, relatively thinner cutaneous tissue C and/or a relatively shallow blood vessel V, e.g., located close to the interface between the cutaneous tissue C and the hypodermis H, preferably decrease the expected perivascular tissue depth for readily observing an anatomical change. There may be a time delay observing anatomical changes that begin at unexpected distances from electromagnetic radiation sensor 100. The delay may last until the anatomical change extends within the observational limits of electromagnetic radiation sensor 100. For example, if anatomical changes over time begin at unexpected depths below the stratum corneum, observing the anatomical change may be delayed until the anatomical change extends to the expected depths below the stratum corneum.

The shapes of emission and detection faces 112 and 122 preferably are related to the spacing distance range between emission and detection waveguides 110 and 120. Preferably, each individual point of emission face 112 is disposed a minimum distance from each individual point of detector face 122, and each individual point of emission face 112 is disposed a maximum distance from each individual point of detector face 122. The minimum and maximum distances preferably correspond to the extremes of the range for the intermediate spacing distance D2. Preferably, the minimum distance is between approximately 2 millimeters and approximately 3.5 millimeters, and the maximum distance preferably is between approximately 4.5 millimeters and approximately 10 millimeters. According to one embodiment, each individual point of emission face 112 is disposed a minimum distance not less than 3 millimeters from each individual point of collection face 122, and each individual point of emission face 112 is disposed a maximum distance not more than 5 millimeters from each individual point of collection face 122. Preferably, the minimum distance is approximately 3.5 millimeters and the maximum distance is approximately 4.5 millimeters. According to other embodiments, each individual point of emission face 112 is spaced from each individual point of collection face 122 such that emitted electromagnetic radiation 102 transitions to collected electromagnetic radiation 106 at a depth of penetration into the Animalia tissue preferably between approximately 1 millimeter and approximately 6 millimeters below the stratum corneum of the skin S. Preferably, the transition between emitted and collected electromagnetic radiation 102 and 106 along individual electromagnetic radiation paths occur at the point of deepest penetration into the Animalia tissue. Emitted and collected electromagnetic radiation 102 and 106 preferably transition in the hypodermis H and may also transition in the dermis of relatively thick cutaneous tissue C. Preferably, emitted and collected electromagnetic radiation 102 and 106 transition approximately 2.5 millimeters to approximately 3 millimeters below the stratum corneum of the skin S.

Figure 7 illustrates a technique for geometrically developing the shape of emission and detection faces 112 and 122 based on the spacing distance range between emission and detection waveguides 110 and 120. According to one embodiment, a boundary 1010 delimits a portion of superficies 1000 for locating emitter face 112 relative to detector face 122. The geometric development of boundary 1010 preferably is based on pairs of circles that are concentric with each individual end face of detection optical fibers 124. Preferably, a radius of the inner circle for each pair corresponds to a minimum distance of the range for the intermediate spacing distance D2 and a radius of the outer circle for each pair corresponds to a maximum distance of the range for the intermediate spacing distance D2. Boundary 1010 preferably is defined by a locus of points that are (1) outside the inner circles; and (2) inside the outer circles. Preferably, emitter face 112 is located within boundary 1010. According to other embodiments, detector face 122 preferably is located within a boundary developed based on the end faces of emission optical fibers 114.

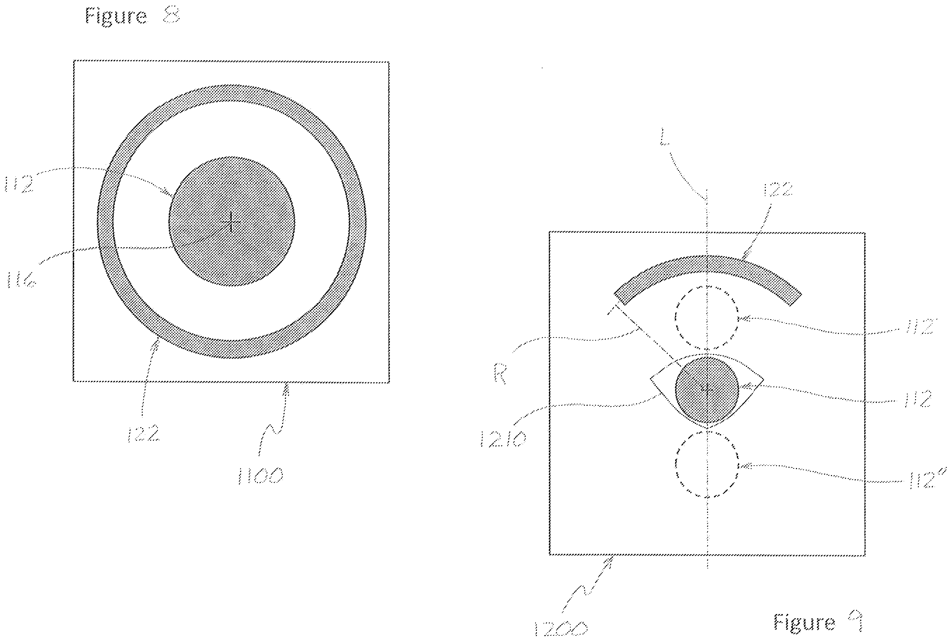

Figures 8-13 show additional examples of superficies that also have suitable geometries for observing anatomical changes over time in the perivascular tissue P. According to one embodiment shown in Figure 8, a superficies 1100 includes emitter face 112 clustered about geometric centroid 116 and an annular detector face 122 that preferably is concentrically disposed about geometric centroid 116. Preferably, annular detector face 122 collects electromagnetic radiation from all directions surrounding emitter face 112. According to other embodiments, detector face 122 preferably includes an incomplete annulus spanning an angular range less than 360 degrees. Preferably, detector face 122 spans an angular range between approximately 25 degrees and approximately 30 degrees.

Figure 9 shows a superficies 1200 illustrating several combinations of geometric variables for emitter face 112 and detector face 122. Preferably, superficies 1200 includes a line of symmetry L that extends through clustered emitter face 112 and arcuate detector face 122. According to one embodiment, emitter face 112 preferably has any shape, e.g., a circle, that is suitable to be disposed inside a boundary 1210, which is similar to boundary 1010 (Figure 7). According to other embodiments, there may be various nominal spacing distances along the line of symmetry L between detector face 122 and emitter face 112, 112' or 112". Accordingly, the radius of curvature R of detector face 122 preferably may be greater than the nominal spacing distance of emitter face 112' from detector face 122, the radius of curvature R of detector face 122 preferably may be substantially equal to the nominal spacing distance of emitter face 112 from detector face 122, or the radius of curvature R of detector face 122 preferably may be less than the nominal spacing distance of emitter face 112" from detector face 122.

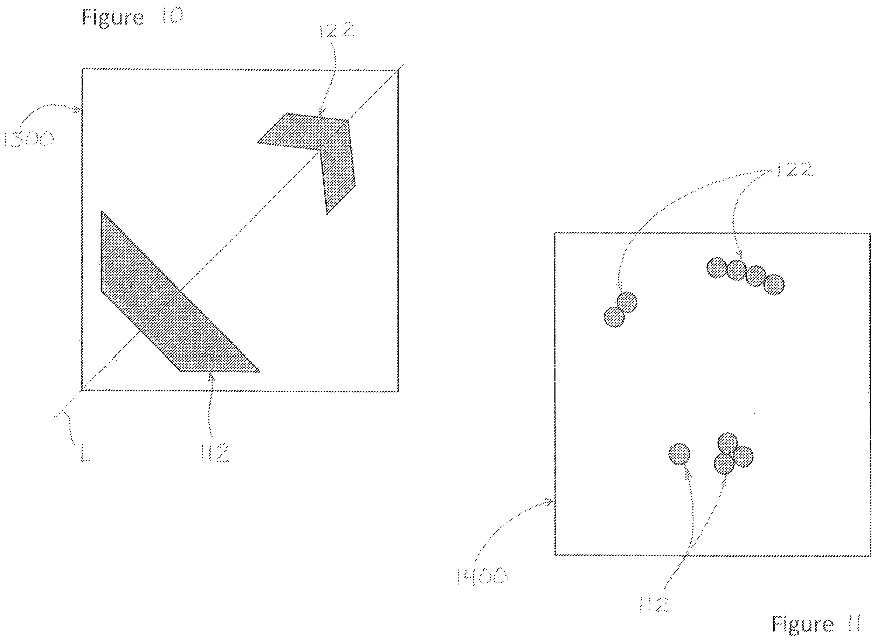

Figure 10 shows a superficies 1300 that illustrates two geometric variables of emitter face 112 from detector face 122. First, the line of symmetry L preferably is angularly oriented with respect to the edges of superficies 1300. In contrast, Figure 9 shows the line of symmetry L perpendicularly oriented with respect to an edge of superficies 1200. Preferably, a diagonal orientation of the line of symmetry L enlarges the range of the spacing distance available between emission and detection waveguides 110 and 120. Second, the shapes of emitter face 112 and/or detector face 122 preferably include polygons. For example, the shape of emitter face 112 is a trapezoid and the shape of detector face 122 is a chevron.

Figure 11 shows a superficies 1400 including emitter and detector faces 112 and 122 that preferably are non-specifically shaped. According to one embodiment, non-specifically shaped emitter and detector faces 112 and 122 preferably are caused by a generally happenstance dispersion of emission and detection optical fibers 114 and 124 in housing 130. According to other embodiments, non-specifically shaped emitter and detector faces 112 and 122 preferably occur because broken fibers are unable to transmit emitted or collected electromagnetic radiation 102 or 106. Preferably, the range of spacing distances between emitter face 112 and detector face 122 for superficies 1400 is generally similar to superficies 1000-1300.

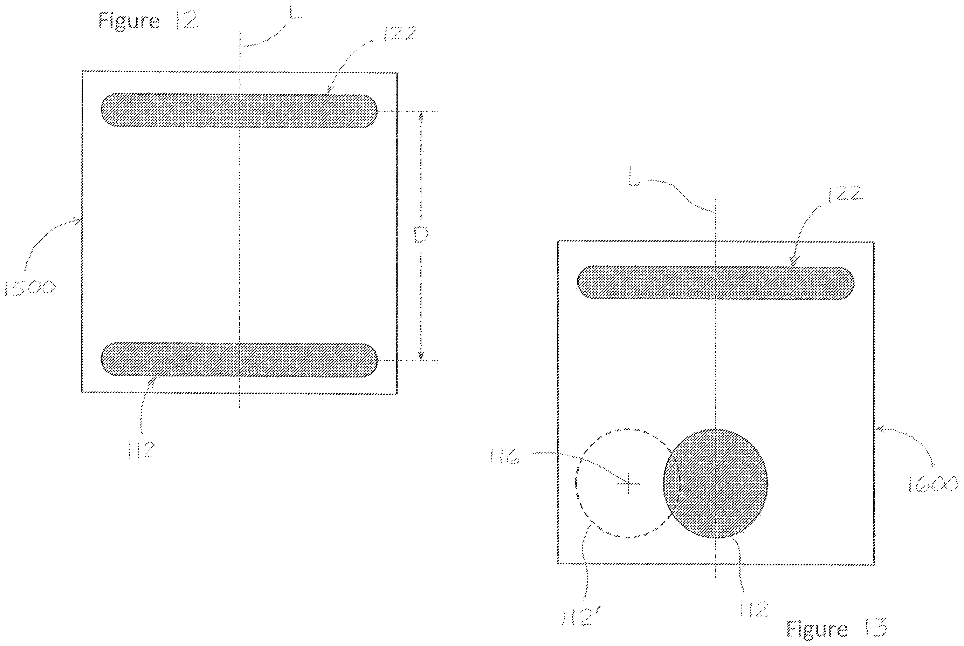

Figure 12 shows a superficies 1500 according to another embodiment including preferably parallel emitter and detector faces 112 and 122. Superficies 1500 preferably includes a line of symmetry L that extends perpendicular to emitter and detector faces 112 and 122. Preferably, the nominal spacing distance D between emission and detection waveguides 110 and 120 is largest when emitter and detector faces 112 and 122 are individually disposed near opposite edges of superficies 1500. According to one embodiment, emitter and detector faces 112 and 122 include bands disposed in parallel straight lines. Accordingly, the perpendicular and diagonal lengths between emitter and detector faces 112 and 122 preferably approximate the minimum and maximum values, respectively, of the spacing distance range between individual points of emitter and detector faces 112 and 122. According to other embodiments, emitter and detector faces 112 and 122 preferably are disposed in parallel arcs. According to other embodiments, emitter and detector faces 112 and 122 preferably are substantially congruent.

Figure 13 shows a superficies 1600 illustrating several combinations of geometric variables for emitter face 112 from detector face 122. According to one embodiment, superficies 1600 includes a line of symmetry L that preferably extends through clustered emitter face 112 and straight-line detector face 122. According to other embodiments, a clustered emitter face 112' preferably is offset from the line of symmetry L. Preferably, the line of symmetry L extends generally perpendicular to a longitudinal axis of straight-line detector 122, and emitter face 112' includes geometric centroid 116 that is laterally displaced with respect to the symmetry L.

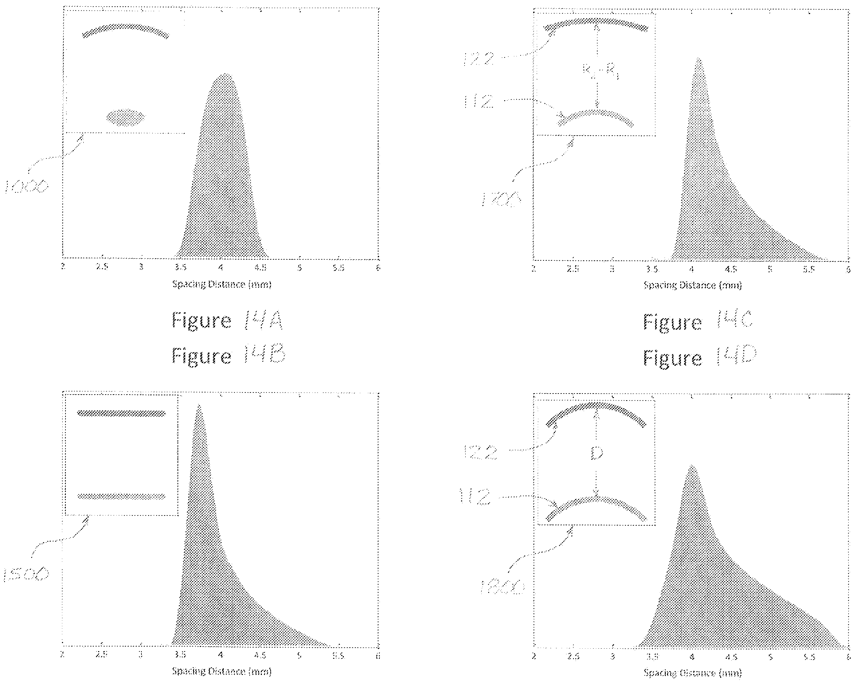

Individual superficies geometries preferably are suitable for observing anatomical changes over time in the perivascular tissue P at various depths below the stratum corneum. As discussed above, the depth below the stratum corneum of the perivascular tissue P at which signals indicative of anatomical changes over time preferably are expected to be observed is at least partially related to the range of spacing distances between emission and detection waveguides 110 and 120. Figures 14A-14D illustrate distributions of the spacing distance ranges for examples of superficies geometries.

Figure 14A shows a distribution of the spacing distance range between individual points of emitter and detector faces 112 and 122 for superficies 1000 (Figure 4) when the radius of curvature R preferably is approximately 4 millimeters. The spacing distances preferably are in a range spanning approximately 1 millimeter, e.g., between approximately 3.5 millimeters and approximately 4.5 millimeters. Preferably, the distribution has a generally symmetrical profile with a mode that is approximately 4 millimeters. As the terminology is used herein, "mode" preferably refers to the most frequently occurring value in a data set, e.g., a set of spacing distances.

Figure 14B shows a distribution of the spacing distance range between individual points of emitter and detector faces 112 and 122 for superficies 1500 (Figure 12) when the nominal spacing distance D preferably is approximately 4 millimeters. Generally all of the spacing distances preferably are in an approximately 2 millimeter range that is between approximately 3.5 millimeters and approximately 5.5 millimeters. Preferably, the distribution overall has an asymmetrical profile; however, a portion of the profile in an approximately 0.3 millimeter range between approximately 3.6 millimeters and approximately 3.9 millimeters is generally symmetrical with a mode that is approximately 3.75 millimeters.

A comparison of the spacing distance distributions shown in Figures 14A and 14B preferably suggests certain relative characteristics of superficies 1000 and 1500 for observing anatomical changes over time in the perivascular tissue P. Comparing Figures 14A and 14B, the magnitude of the spacing distance distribution at the mode for superficies 1500 is greater than for superficies 1000, the range overall is smaller for superficies 1000 than for superficies 1500, and the generally symmetrical portion is smaller for superficies 1500 than for superficies 1000. Accordingly, superficies 1000 and 1500 preferably have certain relative characteristics for observing anatomical changes over time in the perivascular tissue P including: (1) the peak sensitivity of superficies 1000 covers a broader range of depths below the stratum corneum of the skin S than superficies 1500; (2) the peak sensitivity of superficies 1500 is greater in a narrower range of depths below the stratum corneum of the skin S than superficies 1000; and (3) the sensitivity to signals from deeper depths below the stratum corneum of the skin S is greater for superficies 1500 than for superficies 1000. As the terminology is used herein, "peak sensitivity" preferably refers to an interval of spacing distances including the mode of the spacing distances. The interval preferably includes spacing distances having magnitudes that are at least half of the magnitude of the mode.

Figure 14C shows a distribution of the spacing distance range between individual points of emitter and detector faces 112 and 122 for a superficies geometry 1700. Emitter face 112 is generally arcuate with a radius of curvature $R_1$, detector face 122 is generally arcuate with a radius of curvature $R_2$, and emitter and detector faces 112 and 122 are generally concentric with a separation $R_2$-$R_1$ that preferably is approximately 4 millimeters. Preferably, emitter face 112 includes sets of detection optical fiber end faces arranged in individual generally concentric curves, e.g., similar to curve 126. Generally all of the spacing distances preferably are in an approximately 2 millimeter range that is between approximately 3.7 millimeters and approximately 5.7 millimeters. Preferably, the spacing distance distribution has an asymmetrical profile and a mode that is approximately 4.1 millimeters.

A comparison of the spacing distance distributions shown in Figures 14A-14C preferably suggests certain relative characteristics of superficies 1000, 1500 and 1700 for observing anatomical changes over time in the perivascular tissue P. Comparing Figures 14C and 14A, superficies 1700 includes a generally arcuate emitter face 112 whereas superficies 1000 includes a generally clustered emitter face 112, the magnitude of the spacing distance distribution at the mode for superficies 1700 is greater than for superficies 1000, and superficies 1700 includes a larger overall range of spacing distances than superficies 1000. Accordingly, superficies 1700 and 1000 preferably have certain relative characteristics for observing anatomical changes over time in the perivascular tissue P including: (1) the peak sensitivity of superficies 1000 covers a broader range of depths below the stratum corneum of the skin S than superficies 1700; (2) the peak sensitivity of superficies 1700 is greater in a narrower range of depths below the stratum corneum of the skin S than superficies 1000; and (3) the sensitivity to signals from deeper depths below the stratum corneum of the skin S is greater for superficies 1700 than for superficies 1000. Comparing Figures 14C and 14B, superficies 1700 includes emitter and detector faces 112 and 122 disposed in concentric arcs whereas superficies 1500 includes emitter and detector faces 112 and 122 disposed in parallel straight lines, the magnitude of the spacing distance distribution at the mode for superficies 1700 is less than for superficies 1500, and the mode and the range overall of superficies 1700 are shifted toward greater spacing distances than superficies 1000. Accordingly, superficies 1700 and 1500 preferably have certain relative characteristics for observing anatomical changes over time in the perivascular tissue P including, for example, the peak sensitivity is at a greater depth below the stratum corneum of the skin S for superficies 1700 than for superficies 1500.

Figure 14D shows a distribution of the spacing distance range between individual points of emitter and detector faces 112 and 122 for a superficies geometry 1800. Preferably, emitter and detector faces 112 and 122 include parallel arcs with generally equal radii of curvature and a spacing distance D that is approximately 4 millimeters. Generally all of the spacing distances preferably are in an approximately 2.7 millimeter range that is between approximately 3.3 millimeters and approximately 6 millimeters. Preferably, the spacing distance distribution has an asymmetrical profile and a mode that is approximately 4 millimeters.

A comparison of the spacing distance distributions shown in Figures 14A-14D preferably suggests certain relative characteristics of superficies 1000, 1500, 1700 and 1800 for observing anatomical changes over time in the perivascular tissue P. Comparing Figures 14D and 14A, superficies 1800 includes a generally arcuate emitter face 112 whereas superficies 1000 includes a generally clustered emitter face 112. Preferably, superficies 1800 and 1000 share a number of common characteristics including (1) the modes of the spacing distance distributions are approximately equal; (2) the magnitudes of the modes are approximately equal; and (3) the spacing distance distribution profiles between the range minimums and the modes are generally similar. Individual characteristics of superficies 1800 and 1000 preferably include, for example, distinctive spacing distance distribution profiles between the mode and range maximum. According to one embodiment, the spacing distance distribution of superficies 1800 is larger than superficies 1000 at least partially because for the area of arcuate emitter face 112 (superficies 1800) is larger than the area of clustered emitter face 112 (superficies 1000). Superficies 1800 and 1000 preferably have certain relative characteristics for observing anatomical changes over time in the perivascular tissue P including, for example, superficies 1800 is more sensitivity to signals from deeper depths below the stratum corneum of the skin S than superficies 1000. Comparing Figures 14D and 14B, superficies 1800 includes emitter and detector faces 112 and 122 disposed in parallel arcs whereas superficies 1500 includes emitter and detector faces 112 and 122 disposed in parallel straight lines, the magnitude of the spacing distance distribution at the mode is less for superficies 1800 than for superficies 1500 and superficies 1800 includes a larger overall range of spacing distances than superficies 1500. Accordingly, superficies 1800 and 1500 preferably have certain relative characteristics for observing anatomical changes over time in the perivascular tissue P including: (1) the peak sensitivity of superficies 1800 covers a broader range of depths below the stratum corneum of the skin S than superficies 1500; (2) the peak sensitivity of superficies 1500 is greater in a narrower range of depths below the stratum corneum of the skin S than superficies 1800; and (3) the sensitivity to signals from deeper depths below the stratum corneum of the skin S is greater for superficies 1800 than for superficies 1500. Comparing Figures 14D and 14C, superficies 1800 includes emitter and detector faces 112 and 122 disposed in parallel arcs whereas superficies 1700 includes emitter and detector faces 112 and 122 disposed in concentric arcs. Preferably, superficies 1800 and 1700 share a number of common characteristics including (1) the modes of the spacing distance distributions are similar; and (2) the magnitudes of the modes are similar. Individual characteristics of superficies 1800 and 1700 preferably include, for example, distinctive spacing distance distribution profiles on both sides of the mode. According to one embodiment, superficies 1800 includes a larger overall range of spacing distances than superficies 1700. Superficies 1800 and 1700 preferably have certain relative characteristics for observing anatomical changes over time in the perivascular tissue P including, for example, superficies 1800 is more sensitivity to signals from both shallower and deeper depths below the stratum corneum of the skin S than superficies 1700.

Thus, electromagnetic radiation sensor 100 preferably includes a superficies geometry that improves the signal-to-noise ratio of collected electromagnetic radiation 106. Preferably, superficies geometries include suitable relative shapes and spacing distances between emitter and detector faces 112 and 122. Examples of suitable shapes preferably include clusters, arcs, and straight lines. Suitable spacing distances generally correspond with the expected depth below the stratum corneum for the perivascular tissue P at which anatomical changes over time preferably are readily observed. An example of a suitable spacing distance is approximately 4 millimeters for observing anatomical changes at approximately 2.75 millimeters below the stratum corneum.

The inventors also discovered that the topography of superficies 1X00 preferably impacts the signal-to-noise ratio of electromagnetic radiation sensor 100. As the terminology is used herein, "topography" preferably refers to a three-dimensional surface contour and "superficies 1X00" preferably is a generic reference to any suitable superficies of electromagnetic radiation sensor 100. Preferably, superficies 1X00 includes, for example, superficies 1000 (Figure 4 et al.), superficies 1100 (Figure 8), superficies 1200 (Figure 9), superficies 1300 (Figure 10), superficies 1400 (Figure 11), superficies 1500 (Figure 12 et al.), superficies 1600 (Figure 13), superficies 1700 (Figure 14C), and superficies 1800 (Figure 14D). The inventors discovered, *inter alia,* that the signal-to-noise ratio of electromagnetic radiation sensor 100 preferably improves when the topography of superficies 1X00 minimizes gaps or movement with respect to the epidermis of the skin S.

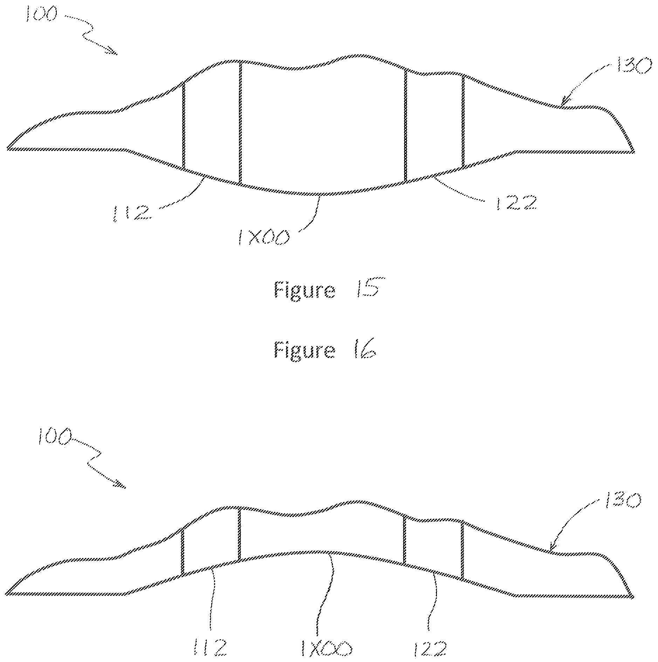

The topography of superficies 1X00 preferably is substantially flat, convex, concave, or a combination thereof. According to one embodiment, superficies 1X00 preferably is substantially flat. For example, superficies 1000 (Figure 4) preferably is a substantially flat plane that overlies the epidermis of the skin S. According to other embodiments, superficies 1X00 preferably includes at least one of a convex superficies 1X00 (Figure 15) and a concave superficies 1X00 (Figure 16) to stretch the epidermis of the skin S. Preferably, the epidermis is stretched when (1) convex superficies 1X00 preferably presses emitter and detector faces 112 and 122 toward the skin S; or (2) the skin S bulges into concave superficies 1X00 toward emitter and detector faces 112 and 122. Pressure along a peripheral edge of concave superficies 1X00 preferably causes the skin S to bulge into concave superficies 1X00. Preferably, stretching the epidermis with respect to superficies 1X00 minimizes relative movement and gaps between electromagnetic radiation sensor 100 and emitter and detector faces 112 and 122.

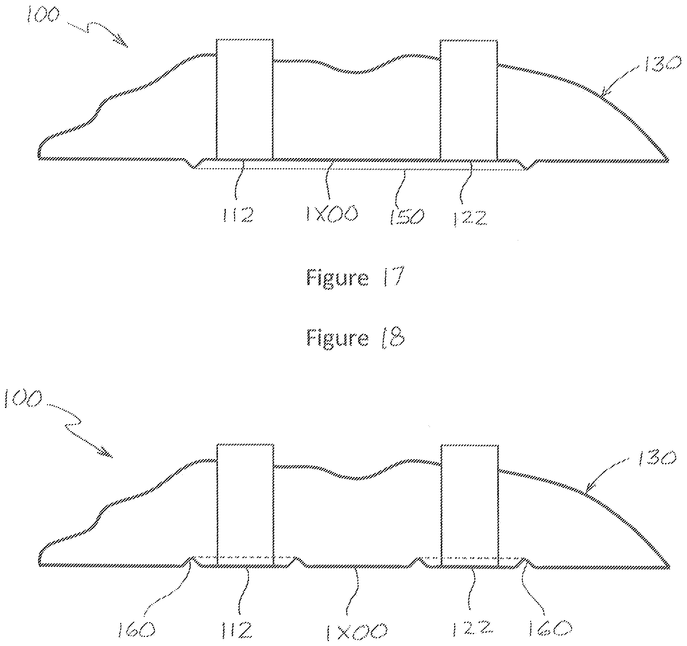

Figures 17 and 18 show additional examples of superficies 1X00 that also have suitable topographies to stretch the epidermis of the skin S. Figure 17 shows a projection 150 extending from superficies 1X00. According to one embodiment, projection 150 preferably cinctures emitter and detector faces 112 and 122. According to other embodiments, separate projections 150 preferably cincture individual emitter and detector faces 112 and 122. Figure 18 shows separate recesses 160 preferably cincturing individual emitter and detector faces 112 and 122. According to other embodiments, a single recess 160 preferably cinctures both emitter and detector faces 112 and 122. Preferably, projection(s) 150 and recess(es) 160 stretch the epidermis with respect to superficies 1X00 to minimize relative movement and gaps between electromagnetic radiation sensor 100 and emitter and detector faces 112 and 122.

Thus, superficies 1X00 preferably include topographies to improve the signal-to-noise ratio of electromagnetic radiation sensor 100. Preferably, suitable topographies that minimize relative movement and gaps between the skin S and emitter and detector faces 112 and 122 include, e.g., flat planes, convex surfaces, concave surfaces, projections and/or recesses.

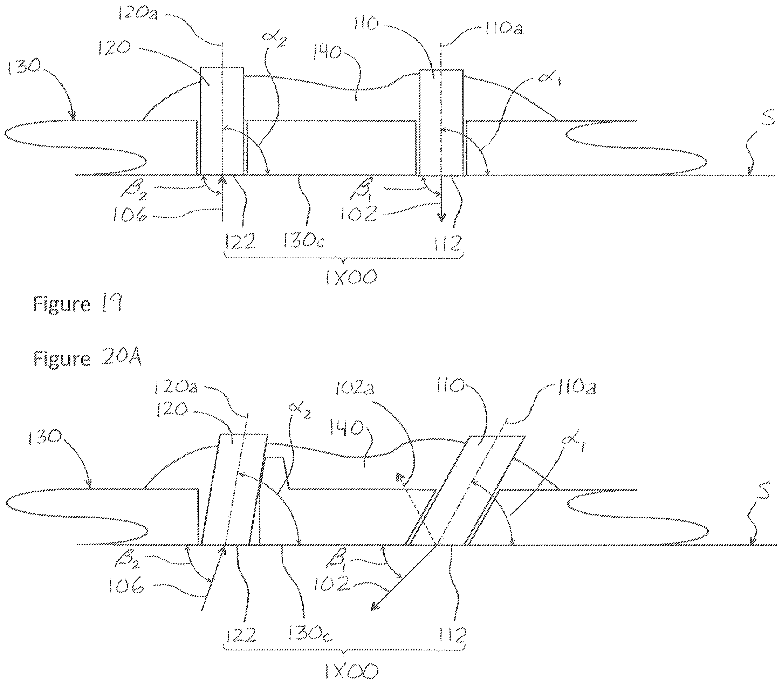

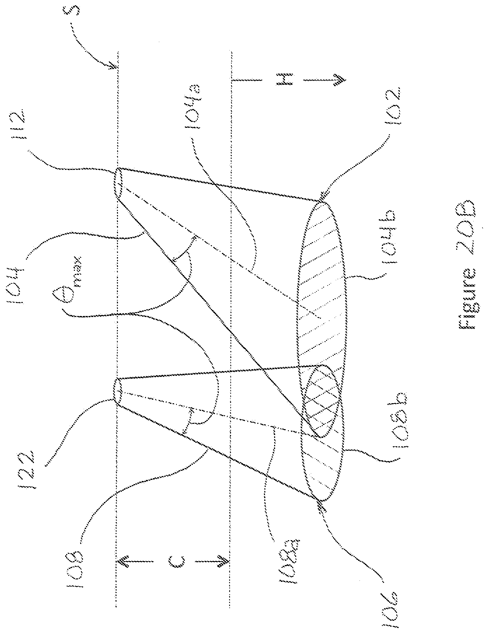

The inventors also discovered, *inter alia,* that angles of intersection between superficies 1X00 and emission and detection waveguides 110 and 120 preferably impact emitted and collected electromagnetic radiation 102 and 106. Figure 19 shows a first embodiment of the angles of intersection, and Figures 20A and 20B show a second embodiment of the angles of intersection. Regardless of the embodiment, emission waveguide 110 transmits electromagnetic radiation generally along a first path 110a to emitter face 112, and detection waveguide 120 transmits electromagnetic radiation generally along a second path 120a from detector face 122.

Superficies 1X00 preferably includes surface 130a and emitter and detector faces 112 and 122. Preferably, first path 110a intersects with superficies 1X00 at a first angle $\alpha_1$ and second path 120a intersects with superficies 1X00 at a second angle $\alpha_2$. In the case of concave or convex superficies 1X00, or superficies 1X00 that include projections 150 or recesses 160, first and second angles $\alpha_1$ and $\alpha_2$ preferably are measured with respect to the tangent to superficies 1X00. Emitted electromagnetic radiation 102 preferably includes at least a part of the electromagnetic radiation that is transmitted along first path 110a, and the electromagnetic radiation transmitted along second path 120a preferably includes at least a part of collected electromagnetic radiation 106. Preferably, emitted electromagnetic radiation 102 exits emitter face 112 within an emission cone 104, and collected electromagnetic radiation 106 enters detector face 122 within an acceptance cone 108. Emission and acceptance cones 104 and 108 preferably include ranges of angles over which electromagnetic radiation is, respectively, emitted by emission waveguide 110 and accepted by detection waveguide 120. Typically, each range has a maximum half-angle $\theta_{max}$ that is related to a numerical aperture NA of the corresponding waveguide as follows: NA $= \eta \sin \theta_{max}$, where $\eta$ is the refractive index of the material that the electromagnetic radiation is entering (e.g., from emission waveguide 110) or exiting (e.g., to detection waveguide 120). The numerical aperture NA of emission or detection optical fibers 114 or 124 typically is calculated based on the refractive indices of the optical fiber core ($\eta_{core}$) and optical fiber cladding ($\eta_{clad}$) as follows:

$$NA = \sqrt{\eta_{core}^2 - \eta_{clad}^2}.$$ Thus, the ability of a waveguide to emit or accept rays from various angles generally is related to material properties of the waveguide. Ranges of suitable numerical apertures NA for emission or detection waveguides 110 or 120 may vary considerably, e.g., between approximately 0.20 and approximately 0.60. According to one embodiment, individual emission or detection optical fibers 114 or 124 preferably have a numerical apertures NA of approximately 0.55. The maximum half-angle $\theta_{max}$ of a cone typically is a measure of an angle between the cone's central axis and conical surface. Accordingly, the maximum half-angle $\theta_{max}$ of emission waveguide 110 preferably is a measure of the angle formed between a central axis 104a and the conical surface of emission cone 104, and the maximum half-angle $\theta_{max}$ of detection waveguide 120 preferably is a measure of the angle formed between a central axis 108a and the conical surface of acceptance cone 108. The direction of central axis 104a preferably is at a first angle $\beta_1$ with respect to superficies 1X00 and the direction of central axis 108a preferably is at a second angle $\beta_2$ with respect to superficies 1X00. Therefore, first angle $\beta_1$ preferably indicates the direction of emission cone 104 and thus also describes the angle of intersection between emitted electromagnetic radiation 102 and superficies 1X00, and second angle $\beta_2$ preferably indicates the direction of acceptance cone 108 and thus also describes the angle of intersection between collected electromagnetic radiation 106 and superficies 1X00. In the case of concave or convex superficies 1X00, or superficies 1X00 that include projections 150 or recesses 160, first and second angles $\beta_1$ and $\beta_2$ preferably are measured with respect to the tangent to superficies 1X00.

Figure 19 shows a generally perpendicular relationship between superficies 1X00 and emission and detection waveguides 110 and 120. The inventors discovered, *inter alia*, if first and second angles $\alpha_1$ and $\alpha_2$ preferably are approximately 90 degrees with respect to superficies 1X00 then (1) first and second angles $\beta_1$ and $\beta_2$ preferably also tend to be approximately 90 degrees with respect to superficies 1X00; (2) emitted electromagnetic radiation 102 preferably is minimally attenuated at the interface between the skin S and emitter face 112; and (3) collected electromagnetic radiation 106 preferably has an improved signal-to-noise ratio. An advantage of having emission waveguide 110 disposed at an approximately 90 degree angle with respect to superficies 1X00 preferably is maximizing the electromagnetic energy that is transferred from along the first path 110a to emitted electromagnetic radiation 102 at the interface between sensor 100 and the skin S. Preferably, this transfer of electromagnetic energy may be improved when internal reflection in waveguide 110 due to emitter face 112 is minimized. Orienting emitter face 112 approximately perpendicular to first path 110a, e.g., cleaving and/or polishing emission optical fiber(s) 114 at approximately 90 degrees with respect to first path 110a, preferably minimizes internal reflection in waveguide 110. Specifically, less of the electromagnetic radiation transmitted along first path 110a is reflected at emitter face 112 and more of the electromagnetic radiation transmitted along first path 110a exits emitter face 112 as emitted electromagnetic radiation 102. Another advantage of having emission waveguide 110 disposed at an approximately 90 degree angle with respect to superficies 1X00 preferably is increasing the depth below the stratum corneum that emitted electromagnetic radiation 102 propagates into the skin S because first angle $\beta_1$ also tends to be approximately 90 degrees when first angle $\alpha_1$ is approximately 90 degrees. Preferably, as discussed above with respect to Figures 2A-2C and 5A-5C, the predominant electromagnetic radiation paths through the skin S are crescent-shaped and the increased propagation depth of emitted electromagnetic radiation 102 may improve the signal-to-noise ratio of collected electromagnetic radiation 106. Thus, according to the first embodiment shown in Figure 19, emission and detection waveguides 110 and 120 preferably are disposed in housing 130 such that first and second paths 110a and 120a are approximately perpendicular to superficies 1X00 for increasing the optical power of emitted electromagnetic radiation 102 and for improving the signal-to-noise ratio of collected electromagnetic radiation 106.

Figures 20A and 20B show an oblique angular relationship between superficies 1X00 and emission and detection waveguides 110 and 120. Preferably, at least one of first and second angles $\alpha_1$ and $\alpha_2$ are oblique with respect to superficies 1X00. First and second angles $\alpha_1$ and $\alpha_2$ preferably are both oblique and inclined in generally similar directions with respect to superficies 1X00. According to one embodiment, the difference between the first and second angles $\alpha_1$ and $\alpha_2$ preferably is between approximately 15 degrees and approximately 45 degrees. Preferably, the first angle $\alpha_1$ is approximately 30 degrees less than the second angle $\alpha_2$. According to other embodiments, first angle $\alpha_1$ ranges between approximately 50 degrees and approximately 70 degrees, and second angle $\alpha_2$ ranges between approximately 75 degrees and approximately 95 degrees. Preferably, first angle $\alpha_1$ is approximately 60 degrees and second angle $\alpha_2$ ranges between approximately 80 degrees and approximately 90 degrees. A consequence of first angle $\alpha_1$ being oblique with respect to superficies 1X00 is that a portion 102a of the electromagnetic radiation transmitted along first path 110a may be reflected at emitter face 112 rather than exiting emitter face 112 as emitted electromagnetic radiation 102. Another consequence is that refraction may occur at the interface between sensor 100 and the skin S because the skin S and the emission and detection waveguides 110 and 120 typically have different refractive indices. Accordingly, first angles $\alpha_1$ and $\beta_1$ would likely be unequal and second angles $\alpha_2$ and $\beta_2$ would also likely be unequal.

Figure 20B illustrates a technique for geometrically interpreting the interplay between emitted electromagnetic radiation 102 and collected electromagnetic radiation 106 when emission and detection waveguides 110 and 120 are obliquely disposed with respect to superficies 1X00. Preferably, emission cone 104 represents the range of angles over which emitted electromagnetic radiation 102 exits emitter face 112, and acceptance cone 108 represents the range of angles over which collected electromagnetic radiation 106 enters detection face 122. Projecting emission and acceptance cones 104 and 108 to a common depth below the stratum corneum of the skin S preferably maps out first and second patterns 104b and 108b, respectively, which are shown with different hatching in Figure 20B. Preferably, the projections of emission and acceptance cones 104 and 108 include a locus of common points where first and second patterns 104b and 108b overlap, which accordingly is illustrated with cross-hatching in Figure 20B. In principle, the locus of common points shared by the projections of emission and acceptance cones 104 and 108 includes tissue that preferably is a focus of electromagnetic radiation sensor 100 for monitoring anatomical changes over time. Accordingly, an advantage of having emission waveguide 110 and/or detection waveguide 120 disposed at an oblique angle with respect to superficies 1X00 preferably is focusing electromagnetic radiation sensor 100 at a particular range of depths below the stratum corneum of the skin S and/or steering sensor 100 in a particular relative direction. In practice, electromagnetic radiation propagating through the skin S is reflected, scattered and otherwise redirected such that there is a low probability of generally straight-line propagation that is contained within the projections of emission and detection cones 104 and 108. Accordingly, Figure 20B preferably is a geometric interpretation of the potential for electromagnetic radiation to propagate to a particular range of depths or in a particular relative direction.

Thus, the angles of intersection between superficies 1X00 and emission and detection waveguides 110 and 120 preferably impact emitted and collected electromagnetic radiation 102 and 106 of electromagnetic radiation sensor 100. Preferably, suitable angles of intersection that improve the optical power of emitted electromagnetic radiation 102, improve the signal-to-noise ratio of collected electromagnetic radiation 106, and/or focus electromagnetic radiation sensor 100 at particular depths/directions include, e.g., approximately perpendicular angles and oblique angles.

The discoveries made by the inventors include, *inter alia,* configurations of an electromagnetic radiation sensor that preferably increase the power of emitted electromagnetic radiation and/or improve the signal-to-noise ratio of collected electromagnetic radiation. Examples of suitable configurations are discussed above including certain superficies geometries, certain superficies topographies, and certain angular orientations of emission and detection waveguides. Preferably, suitable configurations include combinations of superficies geometries, superficies topographies, and/or angular orientations of the waveguides. According to one embodiment, an electromagnetic radiation sensor has a configuration that includes approximately 4 millimeters between waveguides, a convex superficies, and waveguides that intersect the superficies at approximately 90 degrees.

An electromagnetic radiation sensor according to the present disclosure preferably may be used, for example, (1) as an aid in detecting at least one of infiltration and extravasation; (2) to monitor anatomical changes in perivascular tissue; or (3) to emit and collect transcutaneous electromagnetic signals. The discoveries made by the inventors include, *inter alia,* that sensor configuration including geometry (e.g., shape and spacing), topography, and angles of transcutaneous electromagnetic signal emission and detection affect the accurate indications anatomical changes in perivascular tissue, including infiltration/extravasation events. For example, the discoveries made by the inventors include that the configuration of an electromagnetic radiation sensor is related to the accuracy of the sensor for aiding in diagnosing at least one of infiltration and extravasation in Animalia tissue.

Sensors according to the present disclosure preferably are manufactured by certain methods that may vary. Preferably, operations included in the manufacturing method may be performed in certain sequences that also may vary. Examples of a sensor manufacturing method preferably include molding first and second housing portions 130a and 130b. Preferably, superficies 1X00 is molded with first housing portion 130a. At least one emission optical fiber 114 preferably is fed through at least one emission passage 136, which includes emission aperture 136a penetrating superficies 1X00. Preferably, at least one detection optical fiber 124 is fed through at least one detection passage 138, which includes detection aperture 138a also penetrating superficies 1X00. First and second housing portions 130a and 130b preferably are coupled to define interior volume 132. Preferably, emission and detection optical fibers 114 and 124 extend through interior volume 132. Internal portions of emission and detection optical fibers 114 and 124 preferably are fixed with respect to first housing portion 130a. Preferably, internal volume 132 is occluded when filler 140, e.g., epoxy, is injected via fill hole 142. Filler 140 preferably cinctures the internal portions of emission and detection optical fibers 114 and 124 in internal volume 132. Preferably, external portions of emission and detection optical fibers 114 and 124 are cleaved generally proximate superficies 1X00. Cleaving preferably occurs after fixing emission and detection optical fibers 114 and 124 with respect to first housing portion 130a. Preferably, end faces of emission and detection optical fibers 114 and 124 are polished substantially smooth with superficies 1X00. According to one embodiment, each individual point on the end faces of emission optical fibers 114 preferably is disposed a distance not less than 3 millimeters and not more than 5 millimeters from each individual point on the end faces detection optical fibers 124. According to other embodiments, first housing portion 130a preferably is supported with superficies 1X00 disposed orthogonal with respect to gravity when internal portions of emission and detection optical fibers 114 and 124 are fixed with respect to first housing portion 130a. The first and second angles of intersection $\alpha_1$ and $\alpha_2$ between superficies 1X00 and emission and detection optical fibers 114 and 124 therefore preferably are approximately 90 degrees. According to other embodiments, at least one of emission and detection optical fibers 114 and 124 is fixed relative to first housing portion 130 at an oblique angle of intersection with respect to superficies 1X00. According to other embodiments, occluding internal volume 132 preferably includes heating at least one of first housing portion 130a, emission optical fiber 114, and detection optical fiber 124. Preferably, heating facilitates flowing filler 140.

While the present invention has been disclosed with reference to certain embodiments, numerous modifications, alterations, and changes to the described embodiments are possible without departing from the sphere and scope of the present invention, as defined in the appended claims. For example, operation of the sensor may be reversed, e.g., collecting electromagnetic radiation with a waveguide that is otherwise configured for emission as discussed above and emitting electromagnetic radiation with a waveguide that is otherwise configured for detection as discussed above. For another example, relative sizes of the emission and detection waveguides may be reversed, e.g., the emission waveguide may include more optical fibers than the detection waveguide and visa-versa. Accordingly, it is intended that the present invention not be limited to the described embodiments, but that it has the full scope defined by the language of the following claims, and equivalents thereof.

INDUSTRIAL APPLICABILITY

Administering fluids, medications and parenteral nutrition by intravenous infusion therapy is one of the most common procedures in health care. In the United States, approximately 80 percent of patients admitted to hospitals receive intravenous infusion therapy and up to 330,000,000 or more peripheral intravenous administration sets are sold annually. Sensors according to the present disclosure may be used to aid in detecting infusate infiltration and/or extravasation during intravenous infusion therapy. Sensors according to the present disclosure may also be used to monitor blood transfusions or in connection with intravenous infusion therapy for Animalia in addition to human patients.

SEQUENCE LISTING

Not Applicable

**Claims**

1. A sensor (100) to aid in diagnosing at least one of infiltration and extravasation in Animalia tissue, the sensor comprising:

   a housing (130) including a surface (130c) configured to confront an epidermis of the Animalia tissue;
   a first waveguide (110) being configured to transmit a first light signal, the first waveguide

   having an emitter end face (112) configured to emit the first light signal that enters the Animalia tissue;
   guiding the first light signal along a first path intersecting the emitter end face at an approximately 90 degree angle; and
   being partially disposed in the housing;

   a second waveguide (120) being configured to transmit a second light signal, the second light signal including a portion of the first light signal that is at least one of reflected, scattered and redirected from the Animalia tissue, the second waveguide

having a detector end face (!22) configured to collect the second light signal that exits the Animalia tissue;

guiding the second light signal along a second path intersecting the detector end face at an approximately 90 degree angle; and being partially disposed in the housing; and

a substantially smooth superficies (1000) configured to overlie the epidermis, the superficies including the surface, the emitter end face and the detector end face;

**characterised in that**:

each individual point of the emitter end face is disposed a minimum distance not less than 3 millimeters from each individual point of the detector end face, and each individual point of the emitter end face is disposed a maximum distance not more than 5 millimeters from each individual point of the detector end face, to readily observe anatomical changes at depths between approximately 2.5 millimeters and approximately 3 millimeters below the stratum corneum for wavelengths between approximately 650 nanometers and approximately 950 nanometers.

2. The sensor of claim 1 wherein the housing defines an internal volume (132), and each of the first and second waveguides are partially disposed in the internal volume, a filler (140) comprising epoxy being disposed in the internal volume and generally cincturing portions of the first and second waveguides disposed in the internal volume, and the superficies include a substantially smooth superficies configured to overlie the epidermis, wherein the superficies include the surface, the emitter end face, the detector end face, and a façade of the filler.

3. The sensor of claim 1 wherein (i) the first waveguide includes a plurality of emission optical fibers (114), and the emitter end face includes an aggregation of individual end faces of the emission optical fibers; and (ii) the second waveguide includes a plurality of detection optical fibers (124), and the detector end face includes an aggregation of individual end faces of the detection optical fibers.

4. A method of manufacturing a sensor according to any preceding claim to aid in diagnosing at least one of infiltration and extravasation in Animalia tissue, the method comprising:

feeding an emission optical fiber through an emission aperture penetrating a surface configured to confront an epidermis of the Animalia tissue;

feeding a detection optical fiber through a detection aperture penetrating the surface;

coupling first and second housing portions to define an interior volume, the first housing portion including the surface, and the emission and detection optical fibers extending through the interior volume; and

polishing an emitter end face of the emission optical fiber and polishing a detector end face of the detection optical fiber, the emitter and detector end faces being substantially smooth with the surface;

**characterised in that**:

each individual point of the emitter end face is disposed a minimum distance not less than 3 millimeters from each individual point of the detector end face, and each individual point of the emitter end face is disposed a maximum distance not more than 5 millimeters from each individual point of the detector end face, to readily observe anatomical changes at depths between approximately 2.5 millimeters and approximately 3 millimeters below the stratum corneum for wavelengths between approximately 650 nanometers and approximately 950 nanometers.

5. The method of claim 4, comprising injecting epoxy cincturing the emission and detection optical fibers in the interior volume and filling the interior volume.

6. The method of claim 4, wherein feeding the emission optical fiber through the emission aperture includes orienting the emission optical fiber at a first angle with respect to the surface, and feeding the detection optical fiber through the detection aperture includes orienting the detection optical fiber at a second angle with respect to the surface, wherein the first and second angles are approximately 90 degrees.

**Patentansprüche**

1. Sensor (100) zur Unterstützung beim Diagnostizieren von zumindest einer aus Infiltration und Extravasation in Tiergewebe, wobei der Sensor Folgendes umfasst:

ein Gehäuse (130), das eine Oberfläche (130c) umfasst, die konfiguriert ist, einer Epidermis des Tiergewebes zugewandt zu sein;

einen ersten Wellenleiter (110), der konfiguriert ist, ein erstes Lichtsignal zu senden, wobei der erste Wellenleiter

eine Strahler-Endfläche (112) aufweist, die konfiguriert ist, das erste Lichtsignal zu emittieren, das in das Tiergewebe eintritt;

das erste Lichtsignal entlang eines die Strahler-Endfläche in einem ungefähr 90 Grad -Winkel schneidenden, ersten Pfades führt; und

zum Teil in dem Gehäuse angeordnet ist;

einen zweiten Wellenleiter (120), der konfiguriert ist, ein zweites Lichtsignal zu senden, wobei das zweite Lichtsignal einen Abschnitt des ersten Lichtsignals umfasst, der zumindest einer von einem ist, der vom Tiergewebe reflektiert, gestreut und umgeleitet wird, wobei der zweite Wellenleiter

eine Detektor-Endfläche (122) aufweist, die konfiguriert ist, das zweite Lichtsignal, das aus dem Tiergewebe austritt, zu erfassen;

das zweite Lichtsignal entlang eines die Detektor-Endfläche in einem ungefähr 90 Grad -Winkel schneidenden, zweiten Pfades führt; und

zum Teil in dem Gehäuse angeordnet ist; und

eine im Wesentlichen glatte Mantelfläche (1000), die konfiguriert ist, über der Epidermis zu liegen, wobei die Mantelfläche die Oberfläche, die Strahler-Endfläche und die Detektor-Endfläche umfasst;

**dadurch gekennzeichnet, dass**:

jeder einzelne Punkt der Strahler-Endfläche von jedem einzelnen Punkt der Detektor-Endfläche in einer minimalen Entfernung von nicht weniger als 3 Millimeter angeordnet ist, und jeder einzelne Punkt der Strahler-Endfläche von jedem einzelnen Punkt der Detektor-Endfläche in einer maximalen Entfernung von nicht mehr als 5 Millimeter angeordnet ist, um anatomische Änderungen bei Tiefen zwischen ungefähr 2,5 Millimeter und ungefähr 3 Millimeter unterhalb der Hornschicht für Wellenlängen zwischen ungefähr 650 Nanometer und ungefähr 950 Nanometer problemlos wahrzunehmen.

2. Sensor nach Anspruch 1, wobei das Gehäuse ein Innenvolumen (132) definiert, und jeder des ersten und des zweiten Wellenleiters zum Teil im Innenvolumen angeordnet ist, wobei ein Füllstoff (140), der Epoxid umfasst, im Innenvolumen und im Wesentlichen in umgebenden Abschnitten des im Innenvolumen angeordneten, ersten und zweiten Wellenleiters angeordnet ist, und die Mantelflächen eine im Wesentlichen glatte Mantelfläche umfassen, die konfiguriert ist, über der Epidermis zu liegen, wobei die Mantelflächen die Oberfläche, die Strahler-Endfläche, die Detektor-Endfläche und eine Außenseite des Füllstoffs umfassen.

3. Sensor nach Anspruch 1, wobei (i) der erste Wellenleiter eine Vielzahl von Emissionslichtleitfasern (114) umfasst, und die Strahler-Endfläche eine Ansammlung von einzelnen Endflächen der Emissionslichtleitfasern umfasst; und (ii) der zweite Wellenleiter eine Vielzahl von Detektionslichtleitfasern (124) umfasst, und die Detektor-Endfläche eine Ansammlung von einzelnen Endflächen der Detektionslichtleitfasern umfasst.

4. Verfahren zur Herstellung eines Sensors gemäß einem vorangegangenen Anspruch zur Unterstützung beim Diagnostizieren von zumindest einer aus Infiltration und Extravasation in Tiergewebe, wobei das Verfahren Folgendes umfasst:

Einführen einer Emissionslichtleitfaser durch eine Emissionsöffnung hindurch, die eine Oberfläche, die konfiguriert ist, einer Epidermis des Tiergewebes zugewandt zu sein, durchdringt,

Einführen einer Detektionslichtleitfaser durch eine Detektionsöffnung hindurch, welche die Oberfläche durchdringt;

Koppeln des ersten und des zweiten Gehäuseabschnitts, um ein Innenvolumen zu definieren, wobei der erste Gehäuseabschnitt die Oberfläche umfasst, und die Emissions- und die Detektions-Lichtleitfasern sich durch das Innenvolumen hindurch erstrecken; und

Polieren einer Strahler-Endfläche der Emissionslichtleitfaser und Polieren einer Detektor-Endfläche der Detektionslichtleitfaser, wobei die Strahler- und die Detektor-Endfläche im Wesentlichen oberflächenbündig sind;

**dadurch gekennzeichnet, dass**:

jeder einzelne Punkt der Strahler-Endfläche von jedem einzelnen Punkt der Detektor-Endfläche in einer minimalen Entfernung von nicht weniger als 3 Millimeter angeordnet ist, und jeder einzelne Punkt der Strahler-Endfläche von jedem Punkt der Detektor-Endfläche in einer maximalen Entfernung von nicht mehr als 5 Millimeter angeordnet ist, um anatomische Änderungen in Tiefen zwischen ungefähr 2,5 Millimeter und ungefähr 3 Millimeter unterhalb der Hornschicht für Wellenlängen zwischen ungefähr 650 Nanometer und ungefähr 950 Nanometer problemlos wahrzunehmen.

5. Verfahren nach Anspruch 4, umfassend ein Injizieren von Epoxid, das die Emissions- und die Detektions-Lichtleitfaser im Innenvolumen umgibt und das Innenvolumen füllt.

6. Verfahren nach Anspruch 4, wobei ein Einführen der Emissionslichtleitfaser durch die Emissionsöffnung hindurch ein Ausrichten der Emissionslichtleitfaser in einem ersten Winkel mit Bezug auf die Oberfläche umfasst, und ein Einführen der Detektionslichtleitfaser durch die Detektionsöffnung hindurch ein Ausrichten der Detektionslichtleitfaser in einem zweiten Winkel mit Bezug auf die Oberfläche umfasst, wobei der erste und der zweite Winkel ungefähr 90 Grad sind.

## Revendications

1. Capteur (100) permettant le diagnostic d'au moins une infiltration ou une extravasation dans un tissu animal, ce capteur comprenant :

   un boîtier (130) comprenant une surface (130c) conçue pour faire face à un épiderme du tissu animal ;
   un premier guide d'onde (110) étant conçu pour transmettre un premier signal lumineux, le premier guide d'onde :

   comprenant une face d'extrémité émettrice (112) conçue pour émettre le premier signal lumineux qui pénètre dans le tissu animal ;
   guidant le premier signal lumineux le long d'une première trajectoire croisant la face d'extrémité émettrice à un angle d'environ 90 degrés ; et
   étant partiellement disposé dans le boîtier ;
   un deuxième guide d'onde (120) étant conçu pour transmettre un deuxième signal lumineux, le deuxième signal lumineux comprenant une portion du premier signal lumineux qui est au moins réfléchi, diffusé ou redirigé par le tissu animal, le deuxième guide d'onde :

   comprenant une face d'extrémité détectrice (22) conçue pour collecter le deuxième signal lumineux qui sort du tissu animal ;
   guidant le deuxième signal lumineux le long d'une trajectoire croisant la face d'extrémité détectrice à un angle d'environ 90 degrés ; et
   étant partiellement disposé dans le boîtier ; et

   une superficie globalement lisse (1000) conçue pour recouvrir l'épiderme, la superficie comprenant la surface, la face d'extrémité émettrice et la face d'extrémité détectrice ;
   **caractérisé en ce que** :
   chaque point individuel de la face d'extrémité émettrice est disposé à une distance minimum non inférieure à 3 millimètres de chaque point individuel de la face d'extrémité détectrice et chaque point individuel de la face d'extrémité émettrice est disposée à une distance maximum non supérieure à 5 millimètres de chaque point individuel de la face d'extrémité détectrice, afin d'observer facilement des changements anatomiques à des profondeurs entre environ 2,5 millimètres et environ 3 millimètres en dessous du stratum corneum pour des longueurs d'onde entre environ 650 nanomètres et environ 950 nanomètres.

2. Capteur selon la revendication 1, dans lequel le boîtier définit un volume interne (132), et chacun des premier et deuxième guides d'ondes sont partiellement disposés dans le volume interne, une charge (140) comprenant de l'époxy disposé dans un volume interne et les portions englobant de manière générale les premier et deuxième guides d'ondes disposés dans le volume interne, et les superficies comprennent une superficie globalement lisse conçue pour recouvrir l'épiderme, les superficies comprenant la surface, la face d'extrémité émettrice, la face d'extrémité détectrice et une façade de la charge.

3. Capteur selon la revendication 1, dans lequel (i) le premier guide d'onde comprend une pluralité de fibres optiques d'émission (114) et la face d'extrémité émettrice comprend une agrégation de face d'extrémité individuelles des fibres optiques d'émission ; et (ii) le deuxième guide d'onde comprend une pluralité de fibres optiques de détection (124) et la face d'extrémité détectrice comprend une agrégation de faces d'extrémité individuelles des fibres optiques de détection.

4. Procédé de fabrication d'un capteur selon l'une des revendications précédentes permettant le diagnostic d'au moins une infiltration ou une extravasation dans un tissu animal, ce procédé comprenant :

   l'introduction d'une fibre optique d'émission à travers une ouverture d'émission dans une surface conçue pour faire face à un épiderme du tissu animal ;
   l'introduction d'une fibre optique de détection à travers une ouverture de détection dans la surface ;
   le couplage d'une première et d'une deuxième portions de boîtier afin de définir un volume interne, la première portion de boîtier comprenant la surface et les fibres optiques d'émission et de détection s'étendant à travers le volume interne ; et
   le polissage d'une face d'extrémité émettrice de la fibre optique d'émission et le polissage d'une face d'extrémité détectrice de la fibre optique de détection, les faces d'extrémité émettrice et détectrice étant globalement lisses avec la surface ;
   **caractérisé en ce que** :
   chaque point individuel de la face d'extrémité émettrice est disposé à une distance minimum non inférieure à 3 millimètres de chaque point individuel de la face d'extrémité détectrice et chaque point individuel de la face d'extrémité émettrice est disposée à une distance maximum non supérieure à 5 millimètres de chaque point individuel de la face d'extrémité détectrice, afin d'observer facilement des changements anatomiques à des profondeurs entre environ 2,5 millimètres et environ 3 millimètres en dessous du stratum corneum pour des longueurs d'onde entre environ 650 nanomètres et environ 950 nanomètres.

5. Procédé selon la revendication 4, comprenant l'injection d'un époxy entourant les fibres optiques d'émission et de détection dans le volume interne et le remplissage du volume interne.

6. Procédé selon la revendication 4, dans lequel l'introduction de la fibre optique d'émission à travers l'ouverture d'émission comprend l'orientation de la fibre optique d'émission avec un premier angle par rapport à la surface et l'introduction de la fibre optique de détection à travers l'ouverture de détection comprend l'orientation de la fibre optique de détection avec un deuxième angle par rapport à la surface, le premier et le deuxième angles étant d'environ 90 degrés.

Figure 1

Figure 2A

Figure 2B

Figure 2C

Figure 3

Figure 4
Figure 7

Figure 5A

Figure 5B

Figure 5C

Figure 6

Figure 8

Figure 9

Figure 10

Figure 11

EP 2 830 488 B1

Figure 12

Figure 13

28

Figure 14A

Figure 14B

Figure 14C

Figure 14D

Figure 15

Figure 16

100

130

112  IXOO  ISO  122

Figure 17

Figure 18

100

130

160  112  IXOO  122  160

Figure 19

Figure 20A

Figure 20B

Figure 21A

Figure 21B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040086230 A1